# EUROPEAN PATENT SPECIFICATION

(11) **EP 0 797 676 B1**
(45) Date of publication and mention of the grant of the patent: **28.12.2005**
(21) Application number: 95900422.7
(22) Date of filing: 25.10.1994
(51) Int. Cl.: C12N 15/86, C12N 7/01, C12N 5/10, A61K 48/00, C07K 14/47

(54) **RECOMBINANT ADENOVIRAL VECTOR AND METHODS OF USE**
REKOMBINANTE ADENOVIREN-VEKTOR UND VERFAHREN ZUR VERWENDUNG
VECTEUR RECOMBINANT D'ADENOVIRUS ET PROCEDES D'UTILISATION

(30) Priority: 25.10.1993 US 142669; 19.05.1994 US 246006
(43) Date of publication of application: 01.10.1997
(60) Divisional application: 05020924.6
(73) Proprietor: CANJI, Inc., San Diego CA 92121 (US)
(72) Inventor: GREGORY, Richard J., Carlsbad, CA 92008 (US); WILLS, Ken N., Encinitas, CA 92024 (US); MANEVAL, Daniel C., San Diego, CA 92130 (US)
(74) Representative: UEXKÜLL & STOLBERG
(86) International application number: PCT/US1994/012235
(87) International publication number: WO 1995/011984

(56) References cited:
- WO-A-94/24297
- FR-A- 2 688 514
- EMBO JOURNAL., vol.6, no.6, 1987, EYNSHAM, OXFORD GB pages 1733 - 1740 GHOSH-CHOUDHURY, G. ET AL. 'Protein IX a minor component of the human adenovirus capsid is essential for the packaging of full length genomes'
- GENE., vol.13, no.4, 1981, AMSTERDAM NL pages 375 - 386 DIJKEMA, R. ET AL. 'The gene for polypeptide IX of human adenovirus type 7'
- GENE., vol.13, no.4, 1981, AMSTERDAM NL pages 387 - 394 ENGLER J. A. 'The nucleotide sequence of poly peptide IX gene of human adenovirus type 3'
- JOURNAL OF CELLULAR BIOCHEMISTRY, vol.SUP 0, no.17D, 17 March 1993 page 129 ZATLOUKAL, K. ET AL. 'Receptor-mediated cytokine gene delivery to tumor cells for generation of cancer vaccines'
- JOURNAL OF CELLULAR BIOCHEMISTRY, vol.SUP 0, no.18A, 15 January 1994 page 237 GREGORY, R. J. ET AL. 'Tumor suppressor gene therapy of cancer: adenoviral mediated gene transfer of p53 cDNA into human tumor cell lines'
- CANCER GENE THERAPY, vol.1, no.2, June 1994 pages 107 - 112 SHEWACH, D.S. ET AL. 'Enhanced cytotoxicity of antiviral drugs mediated by adenovirus directed transfer of the herpes simplex virus thymidine kinase gene in rat glioma cells'
- PROCEEDINGS OF THE NATIONAL ACADEMY OF SCIENCES OF USA., vol.87, November 1990, WASHINGTON US pages 8746 - 8750 VENKATESH, L. K. ET AL. 'Selective induction of cytotoxicity to human cells expressing human immunodeficiency virus type 1 tat by a conditionnally cytotoxic adenovirus vector'

## Description

This application is a continuation-in-part of U.S. Serial No. 08/233,777, filed May 19, 1994, which is a continuation-in-part of U.S. Serial No. 08/142,669 filed October 25, 1993.

Throughout this application, various publications are referred to by citations within parentheses and in the bibliographic description, immediately preceding the claims.

Production of recombinant adenoviruses useful for gene therapy requires the use of a cell line capable of supplying in *trans* the gene products of the viral E1 region which are deleted in these recombinant viruses. At present the only useful cell line available is the 293 cell line originally described by Graham *et al*. in 1977. 293 cells contain approximately the left hand 12% (4.3 kb) of the adenovirus type 5 genome (Aiello (1979) and Spector. (1983)).

Adenoviral vectors currently being tested for gene therapy applications typically are deleted for Ad2 or Ad5 DNA extending from approximately 400 base pairs from the 5' end of the viral genome to approximately 3.3 kb from the 5' end, for a total E1 deletion of 2.9 kb. Therefore, there exists a limited region of homology of approximately 1 kb between the DNA sequence of the recombinant virus and the Ad5 DNA within the cell line. This homology defines a region of potential recombination between the viral and cellular adenovirus sequences. Such a recombination results in a phenotypically wild-type virus bearing the Ad5 E1 region from the 293 cells. This recombination event presumably accounts for the frequent detection of wild-type adenovirus in preparations of recombinant virus and has been directly demonstrated to be the cause of wild-type contamination of the Ad2 based recombinant virus Ad2/CFTR-1 (Rich *et al*. (1993)).

Due to the high degree of sequence homology within the type C adenovirus subgroup such recombination is likely to occur if the vector is based on any group C adenovirus (types 1, 2, 5, 6).

In small scale production of recombinant adenoviruses, generation of contaminating wild-type virus can be managed by a screening process which discards those preparations of virus found to be contaminated. As the scale of virus production grows to meet expected demand for genetic therapeutics, the likelihood of any single lot being contaminated with a wild-type virus also will rise as well as the difficulty in providing non-contaminated recombinant preparations.

There will be over one million new cases of cancer diagnosed this year, and half that number of cancer-related deaths (American Cancer Society, 1993). p53 mutations are the most common genetic alteration associated with human cancers, occurring in 50-60% of human cancers (Hollstein *et al*. (1991); Bartek *et al*. (1991); Levine (1993)). The goal of gene therapy in treating p53 deficient tumors, for example, is to reinstate a normal, functional copy of the wild-type p53 gene so that control of cellular proliferation is restored. p53 plays a central role in cell cycle progression, arresting growth so that repair or apoptisis can occur in response to DNA damage. Wild-type p53 has recently been identified as a necessary component for apoptosis induced by irradiation or treatment with some chemotherapeutic agents (Lowe *et al*. (1993) A and B). Due to the high prevalence of p53 mutations in human tumors, it is possible that tumors which have become refractory to chemotherapy and irradiation treatments may have become so due in part to the lack of wild-type p53. By resupplying functional p53 to these tumors, it is reasonable that they now are susceptible to apoptisis normally associated with the DNA damage induced by radiation and chemotherapy.

One of the critical points in successful human tumor suppressor gene therapy is the ability to affect a significant fraction of the cancer cells. The use of retroviral vectors has been largely explored for this purpose in a variety of tumor models. For example, for the treatment of hepatic malignancies, retroviral vectors have been employed with little success because these vectors are not able to achieve the high level of gene transfer required for *in vivo* gene therapy (Huber, B.E. et al., 1991; Caruso M. et al., 1993).

To achieve a more sustained source of virus production, researchers have attempted to overcome the problem associated with low level of gene transfer by direct injection of retroviral packaging cell lines into solid tumors (Caruso, M. et al., 1993; Ezzidine, Z.D. et al., 1991; Culver, K.W. et al., 1992). However, these methods are unsatisfactory for use in human patients because the method is troublesome and induces an inflammatory response against the packaging cell line in the patient. Another disadvantage of retroviral vectors is that they require dividing cells to efficiently integrate and express the recombinant gene of interest (Huber, B.E. 1991). Stable integration into an essential host gene can lead to the development or inheritance of pathogenic diseased states.

Recombinant adenoviruses have distinct advantages over retroviral and other gene delivery methods (for review, see Siegfried (1993)). Adenoviruses have never been shown to induce tumors in humans and have been safely used as live vaccines (Straus (1984)). Replication deficient recombinant adenoviruses can be produced by replacing the E1 region necessary for replication with the target gene. Adenovirus does not integrate into the human genome as a normal consequence of infection, thereby greatly reducing the risk of insertional mutagenesis possible with retrovirus or adeno-associated viral (AAV) vectors. This lack of stable integration also leads to an additional safety feature in that the transferred gene effect will be transient, as the extrachromosomal DNA will be gradually lost with continued division of normal cells. Stable, high titer recombinant adenovirus can be produced at levels not achievable with retrovirus or AAV, allowing enough material to be produced to treat a large patient population. Moreover, adenovirus vectors are capable of highly efficient *in vivo* gene transfer into a broad range of tissue and tumor cell types. For example, others have shown that adenovirus mediated gene delivery has a strong potential for gene therapy for diseases such as cystic fibrosis (Rosenfeld *et al*. (1992); Rich *et al*. (1993)) and α₁-antitrypsin deficiency (Lemarchand *et al*. (1992)). Although other alternatives for gene delivery, such as cationic liposome/DNA complexes, are also currently being explored, none as yet appear as effective as adenovirus mediated gene delivery.

As with treating p53 deficient tumors, the goal of gene therapy for other tumors is to reinstate control of cellular proliferation. In the case of p53, introduction of a functional gene reinstates cell cycle control allowing for apoptotic cell death induced by therapeutic agents. Similarly, gene therapy is equally applicable to other tumor suppressor genes which can be used either alone or in combination with therapeutic agents to control cell cycle progression of tumor cells and/or induce cell death. Moreover, genes which do not encode cell cycle regulatory proteins, but directly induce cell death such as suicide genes or, genes which are directly toxic to the cell can be used in gene therapy protocols to directly eliminate the cell cycle progression of tumor cells.

Regardless of which gene is used to reinstate the control of cell cycle progression, the rationale and practical applicability of this approach is identical. Namely, to achieve high efficiencies of gene transfer to express therapeutic quantities of the recombinant product. The choice of which vector to use to enable high efficiency gene transfer with minimal risk to the patient is therefore important to the level of success of the gene therapy treatment.

Thus, there exists a need for vectors and methods which provide high level gene transfer efficiencies and protein expression which provide safe and effective gene therapy treatments. The present invention satisfies this need and provides related advantages as well.

### SUMMARY OF THE INVENTION

This invention provides a recombinant adenovirus expression vector characterized by a total deletion of the adenoviral protein IX DNA and having a gene encoding a foreign protein or a functional fragment or mutant thereof.

Thus, for example, the adenoviral vector of this invention can contain a foreign gene for the expression of a protein effective in regulating the cell cycle, such as p53, Rb, or mitosin, or in inducing cell death, such as the conditional suicide gene thymidine kinase. (The latter must be used in conjunction with a thymidine kinase metabolite in order to be effective).

### BRIEF DESCRIPTION OF THE FIGURES

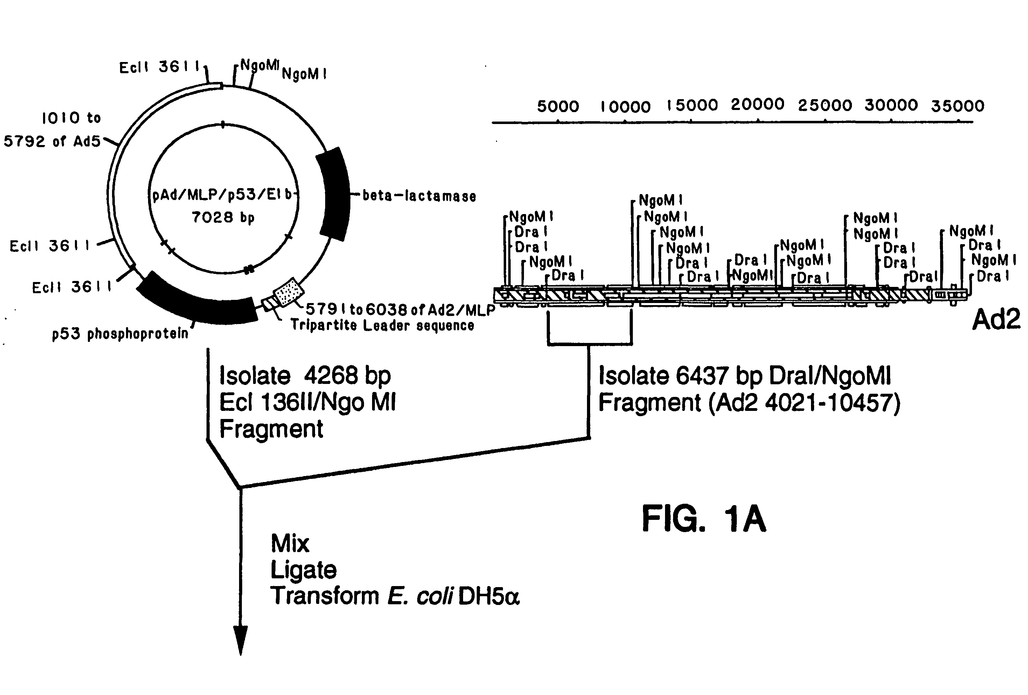

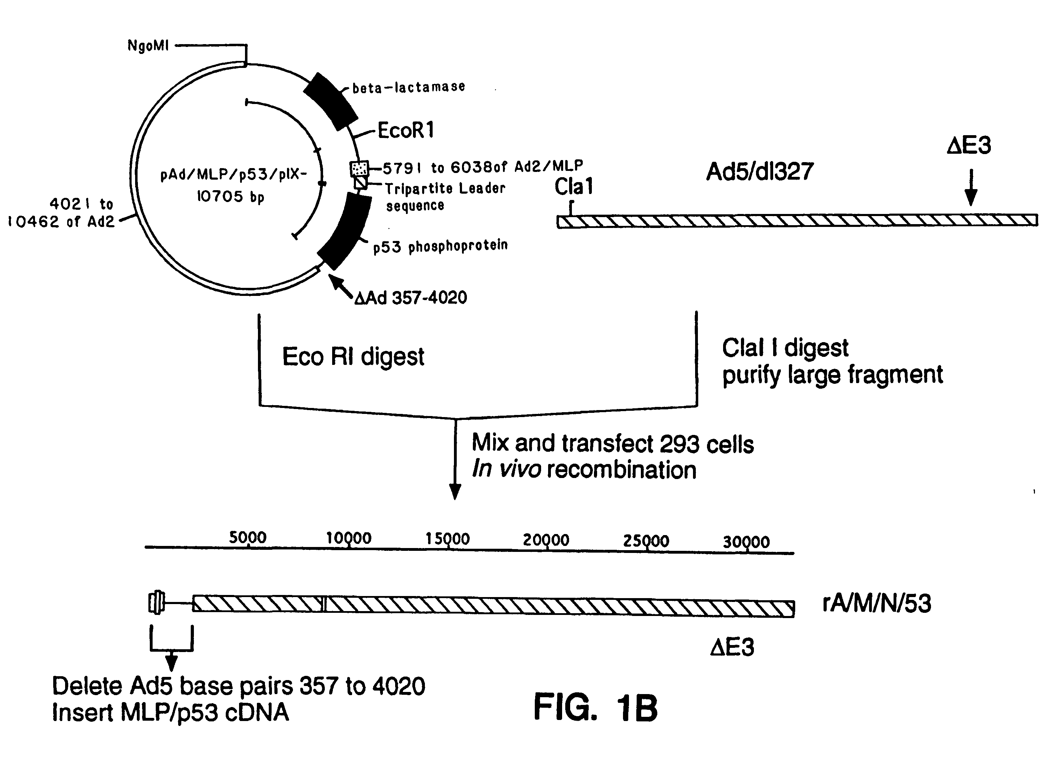

Figure 1 shows a recombinant adenoviral vector of this invention. This construct was assembled as shown in Figure 1. The resultant virus bears a 5' deletion of adenoviral sequences extending from nucleotide 356 to 4020 and eliminates the E1a and E1b genes as well as the entire protein IX coding sequence, leaving the polyadenylation site shared by the E1b and pIX genes intact for use in terminating transcription of any desired gene.

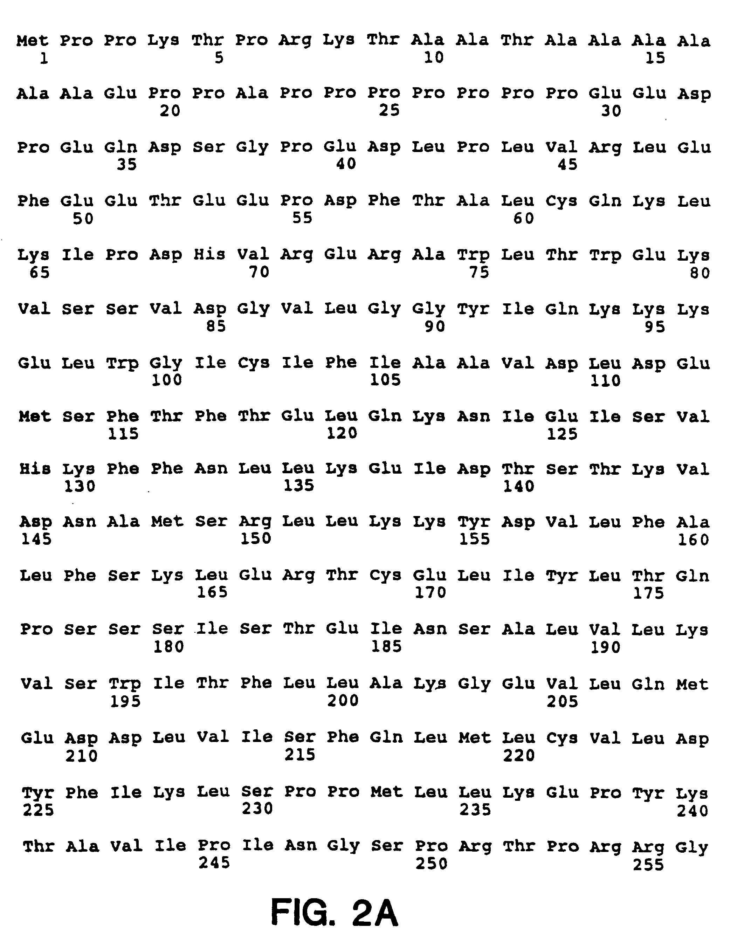

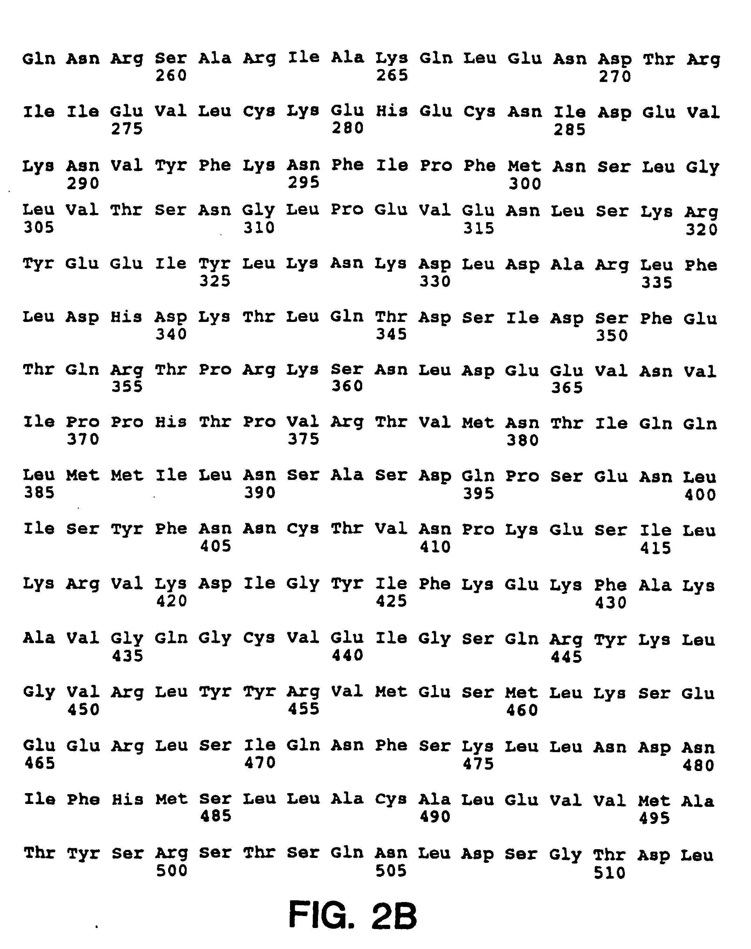

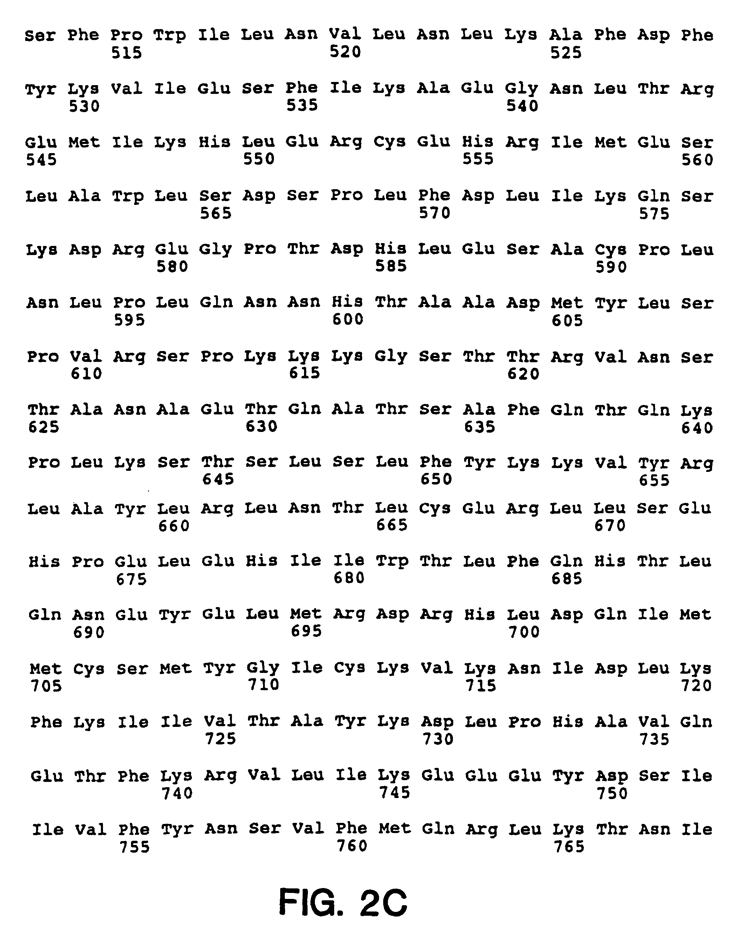

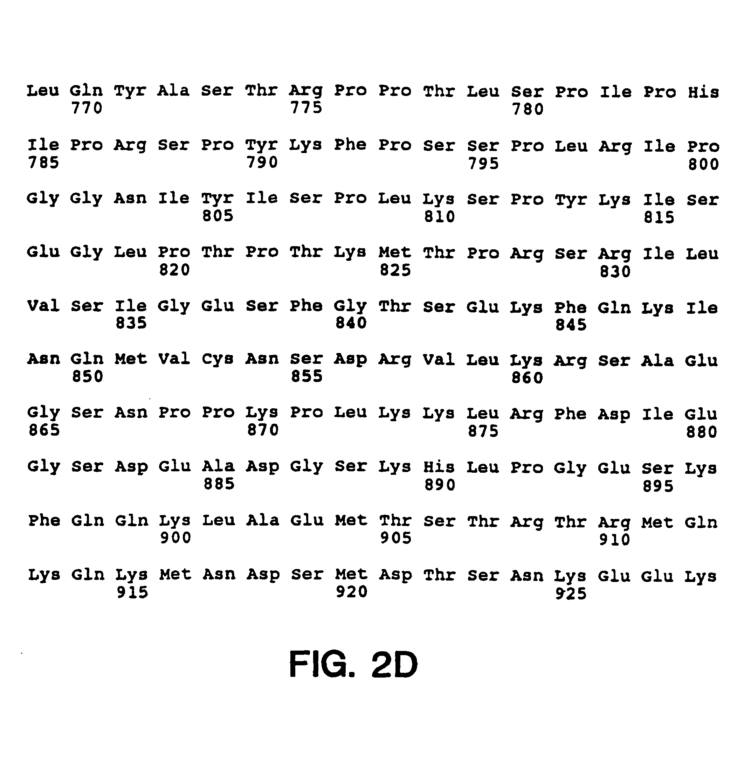

Figure 2 shows the amino acid sequence of p110^{RB}.

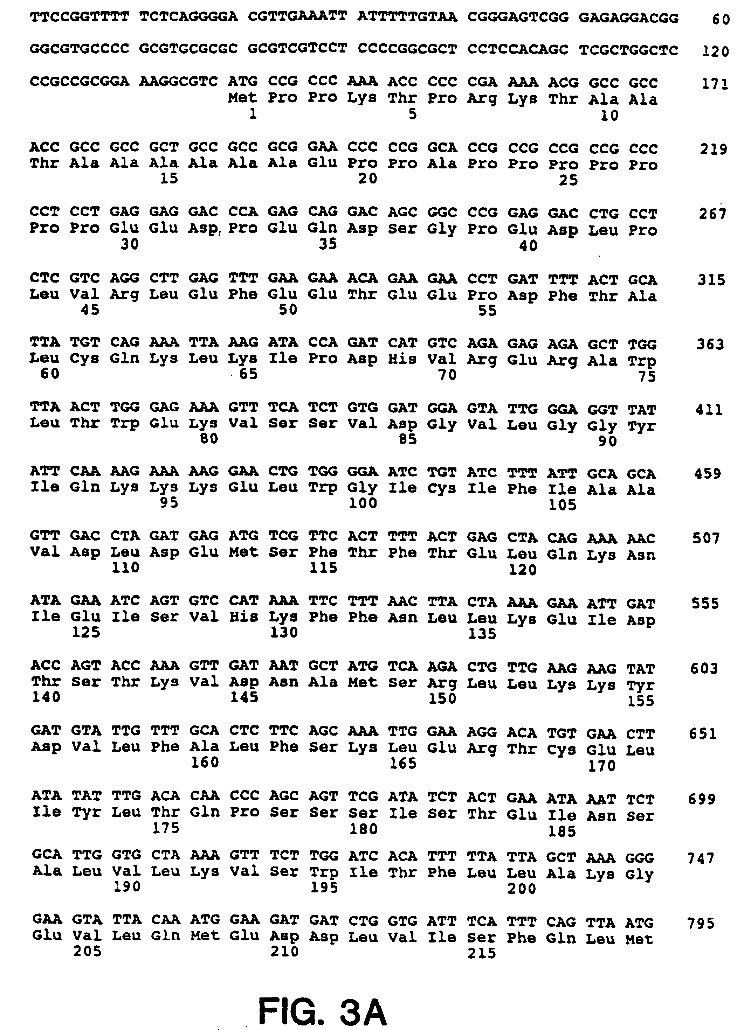

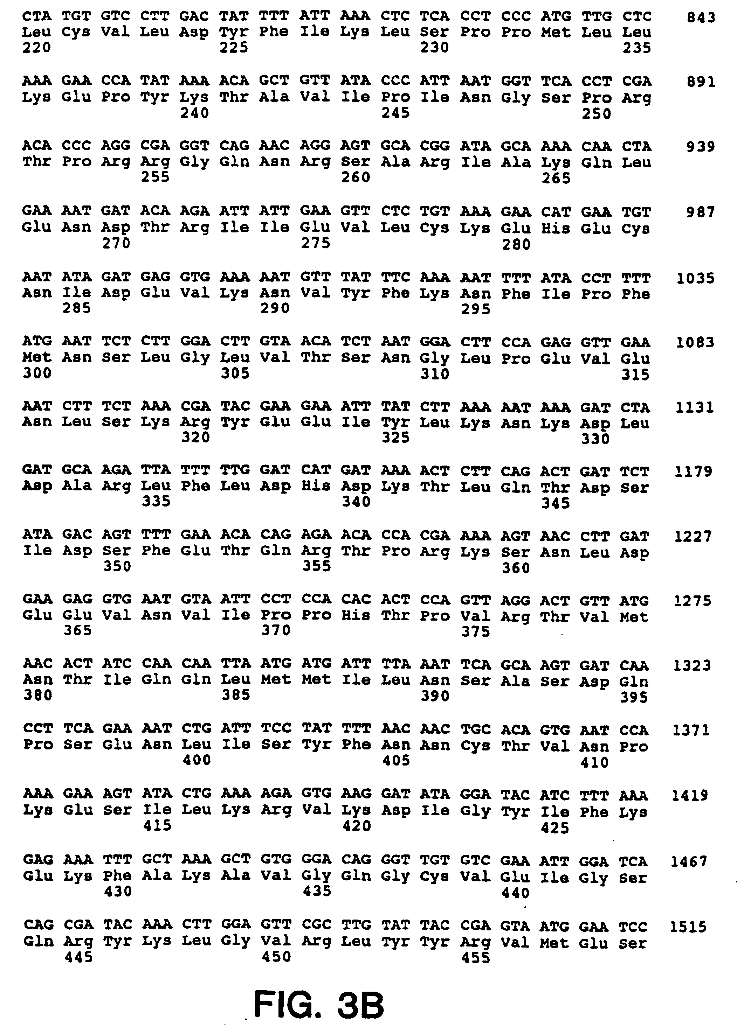

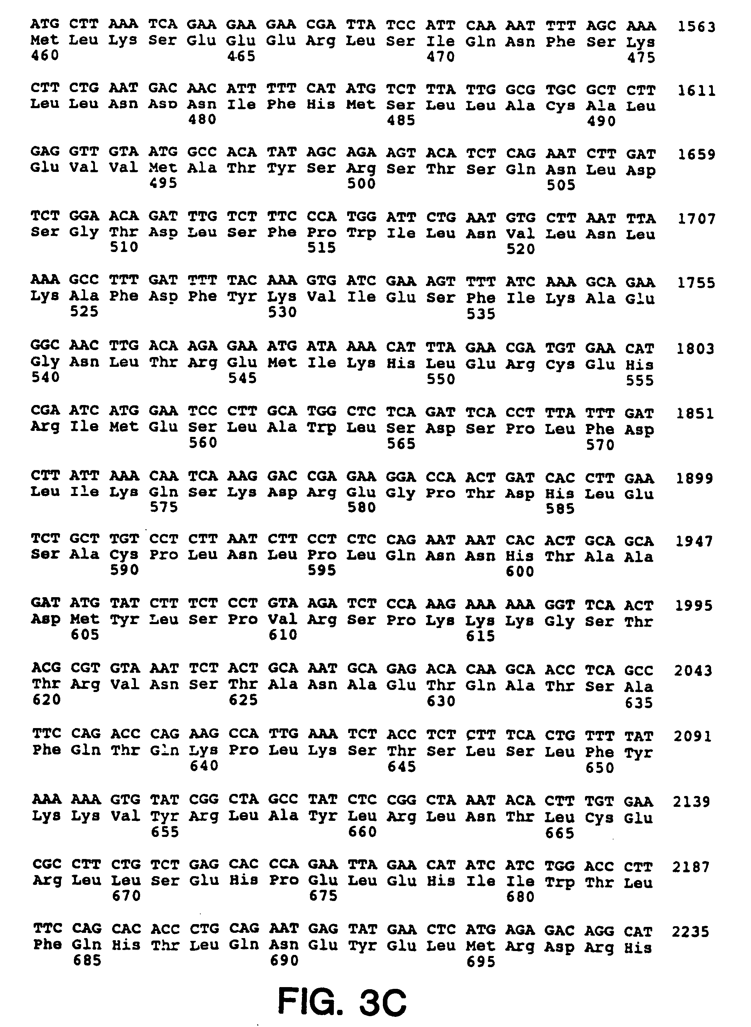

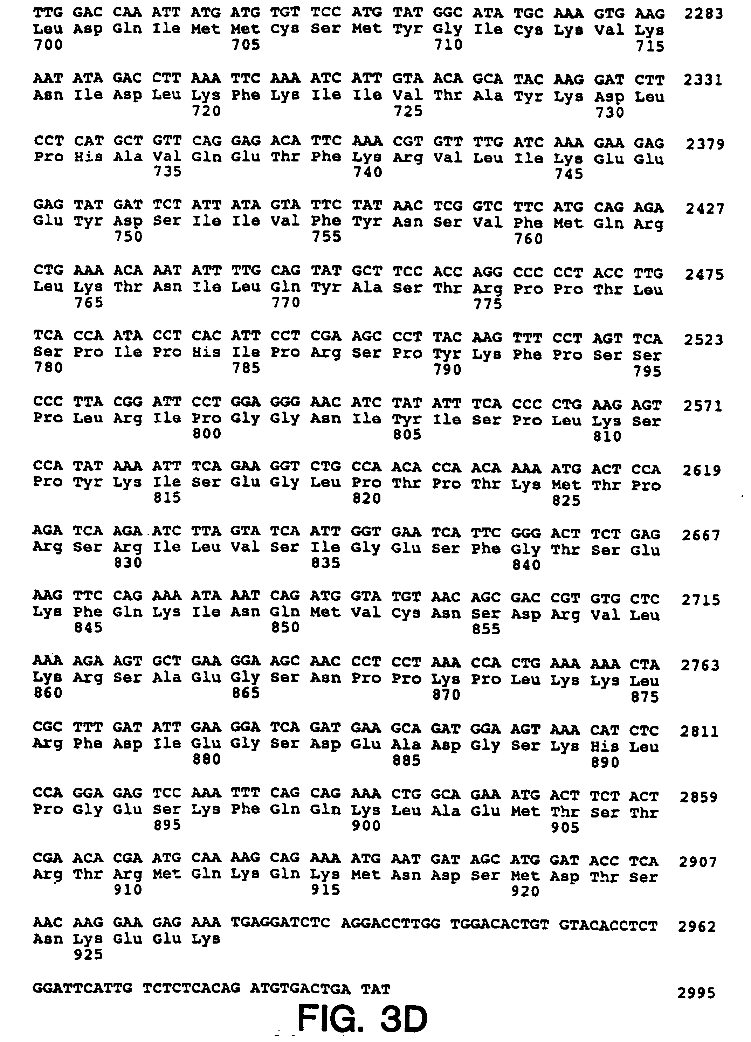

Figure 3 shows a DNA sequence encoding a retinoblastoma tumor suppressor protein.

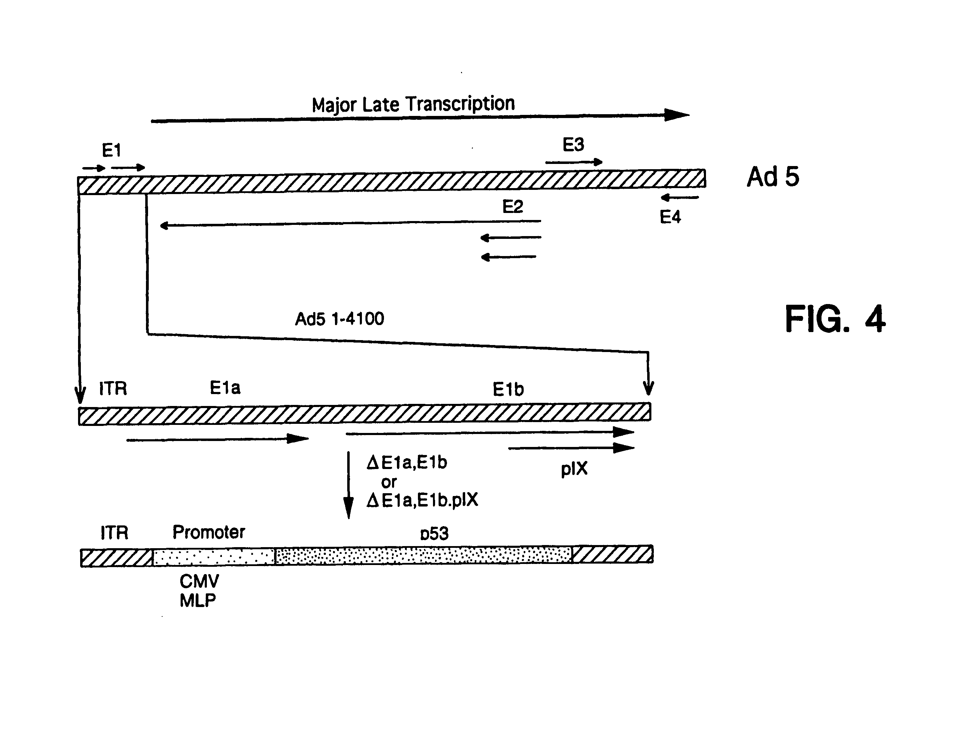

Figure 4 shows schematic of recombinant p53/adenovirus constructs within the scope of this invention. The p53 recombinants are based on Ad 5 and have had the E1 region of nucleotides 360-3325 replaced with a 1.4 kb full length p53 cDNA driven by the Ad 2 MLP (A/M/53) or human CMV (A/C/53) promoters followed by the Ad 2 tripartite leader cDNA. The control virus A/M has the same Ad 5 deletions as the A/M/53 virus but lacks the 1.4 kb p53 cDNA insert. The remaining E1b sequence (705 nucleotides) have been deleted to create the protein IX deleted constructs A/M/N/53 and A/C/N/53. These constructs also have a 1.9 kb Xba I deletion within adenovirus type 5 region E3.

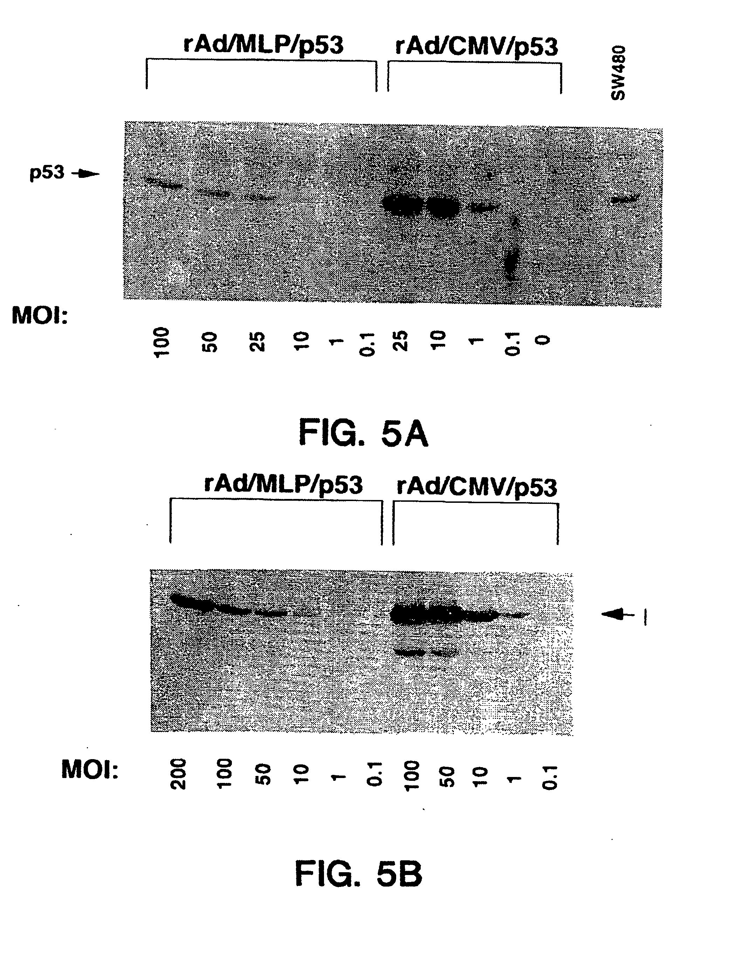

Figures 5A and 5B show p53 protein expression in tumor cells infected with A/M/53 and A/C/53. Figure 5A) Saos-2 (osteosarcoma) cells were infected at the indicated multiplicities of infection (MOI) with either the A/M/53 or A/C/53 purified virus and harvested 24 hours later. The p53 antibody pAb 1801 was used to stain immunoblots of samples loaded at equal total protein concentrations. Equal protein concentration of SW480 cell extracts, which overexpress mutant p53 protein, were used as a marker for p53 size. "O" under the A/C/53 heading indicates a mock infection, containing untreated Saos-2 lysate. Figure 5B) Hep 3B (hepatocellular carcinoma) cells were infected with the A/M/53 or A/C/53 virus at the indicated MOI and analyzed as in part A.) The arrow indicates the position of the p53 protein.

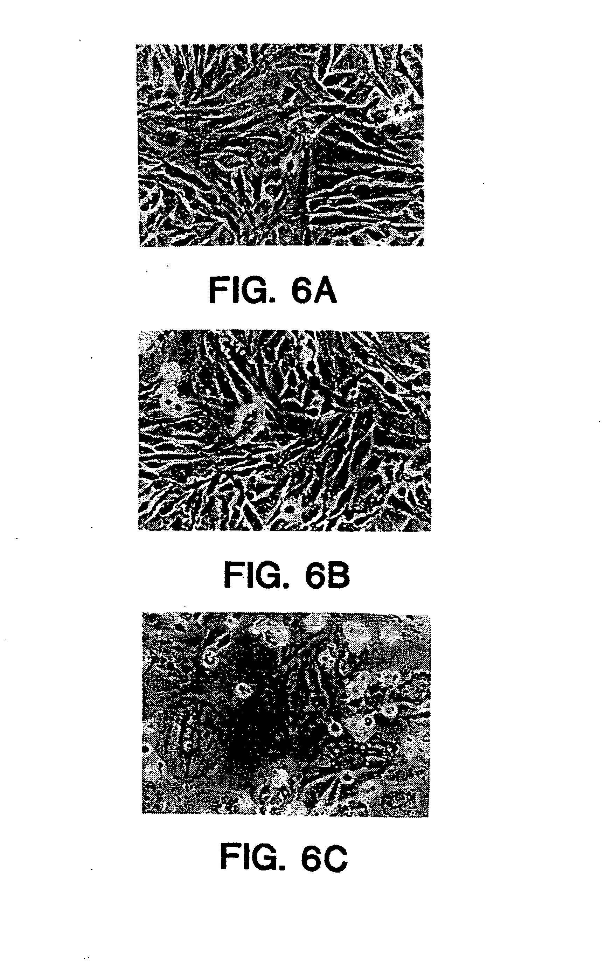

Figures 6A through 6C show p53 dependent Saos-2 morphology change. Subconfluent (1 x 10⁵ cells/10 cm plate) Saos-2 cells were either uninfected (A), infected at an MOI = 50 with (B) the control A/M virus or (C) the A/C/53 virus. The cells were photographed 72 hours post-infection.

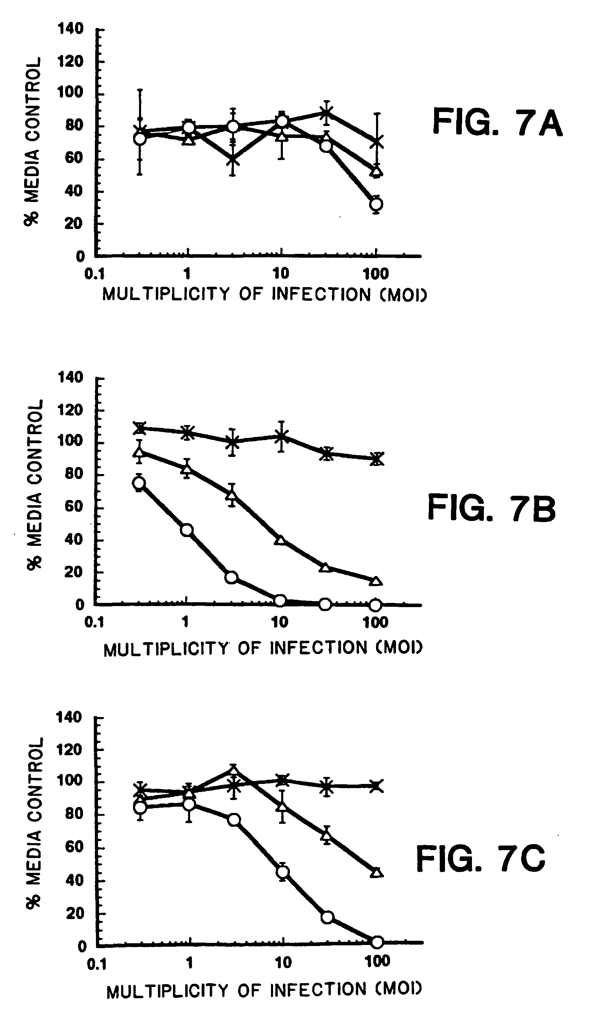

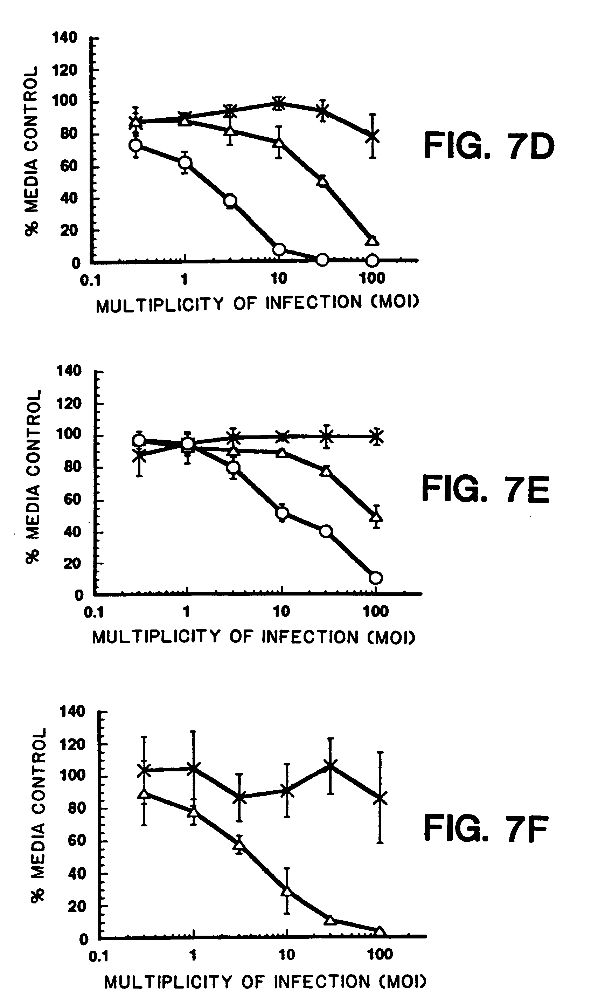

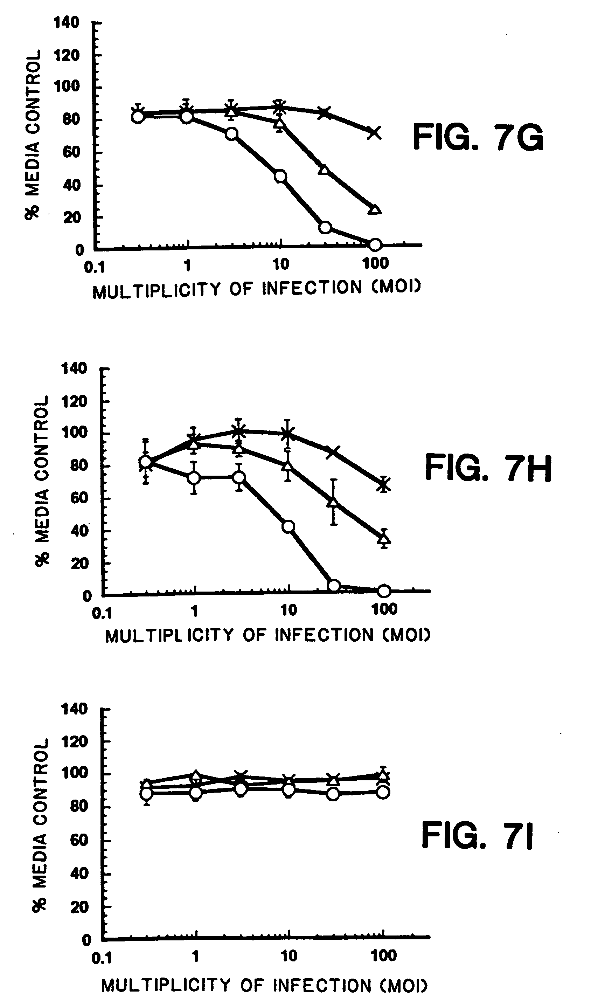

Figure 7 shows p53 dependent inhibition of DNA synthesis in human tumor cell lines by A/M/N/53 and A/C/N/53. Nine different tumor cell lines were infected with either control adenovirus A/M (-x-x-), or the p53 expressing A/M/N/53 (-Δ-Δ-), or A/C/N/53 (-O-O-) virus at increasing MOI as indicated. The tumor type and p53 status is noted for each cell line (wt = wild type, null = no protein expressed, mut = mutant protein expressed). DNA synthesis was measured 72 hours post-infection as described below in Experiment No. II. Results are from triplicate measurements at each dose (mean+/- SD), and are plotted as % of media control versus MOI. * H69 cells were only tested with A/M and A/M/N/53 virus.

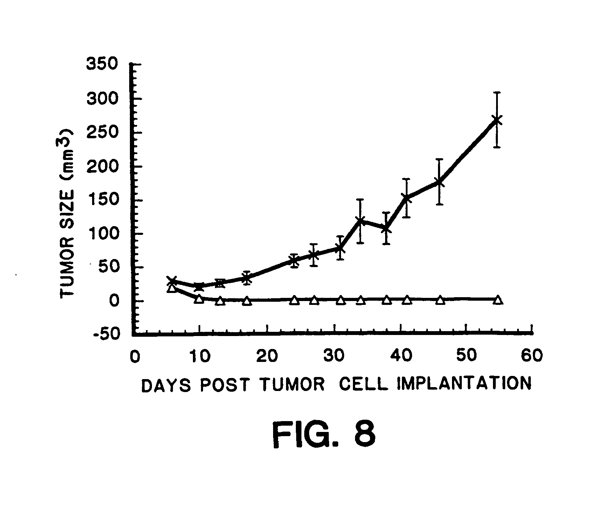

Figure 8 shows tumorigenicity of p53 infected Saos-2 cells in nude mice. Saos-2 cells were infected with either the control A/M virus or the p53 recombinant A/M/N/53 at MOI = 30. Treated cells were injected subcutaneously into the flanks of nude mice, and tumor dimensions were measured (as described in Experiment No. II) twice per week for 8 weeks. Results are plotted as tumor size versus days post tumor cell implantation for both control A/M (-x-x-) and A/M/N/53 (-Δ-Δ-) treated cells. Error bars represent the mean tumor size =/- SEM for each group of 4 animals at each time point.

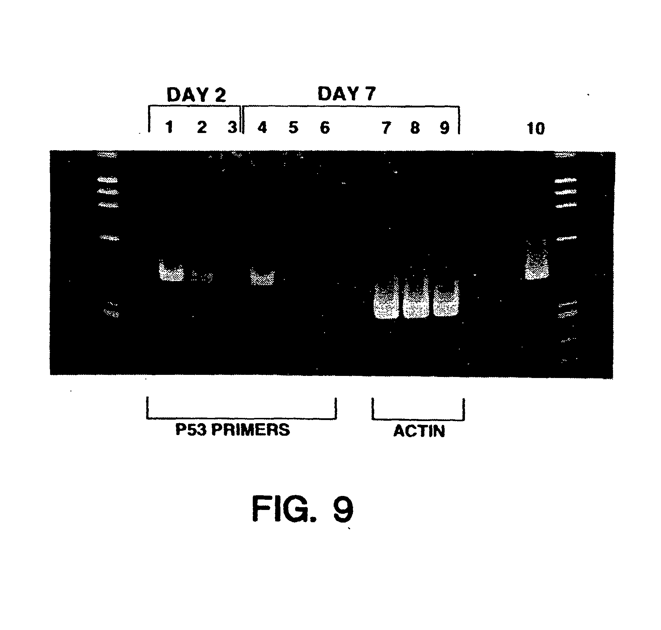

Figure 9 is expression of rAd/p53 RNA in established tumors. H69 (SCLC) cells were injected subcutaneously into nude mice and allowed to develop tumors for 32 days until reaching a size of approximately 25-50 mm³. Mice were randomized and injected peritumorally with 2 x 10⁹ pfu of either control A/C/β-gal or A/C/53 virus. Tumors were excised 2 and 7 days post injection, and polyA RNA was prepared from each tumor sample. RT-PCR was carried out using equal RNA concentrations and primers specific for recombinant p53 message. PCR amplification was for 30 cycles at 94°C 1 min., 55°C 1.5 min., 72°C 2 min., and a 10 min., 72°C final extension period in an Omnigen thermalcycler (Hybaid). The PCR primers used were a 5' Tripartite Leader cDNA (5' - CGCCACCGAGGGACCTGAGCGAGTC-3') and a 3' p53 primer (5' - TTCTGGGAAGGGACAGAAGA-3'). Lanes 1, 2, 4, and 5 are p53 treated samples excised at day 2 or 7 as indicated. Lanes 3 and 6 are from β-gal treated tumors. Lanes 7,8, and 9 are replicates of lanes 4,5, and 6 respectively, amplified with actin primers to verify equal loading. Lane 10 is a positive control using a tripartite/p53 containing plasmid.

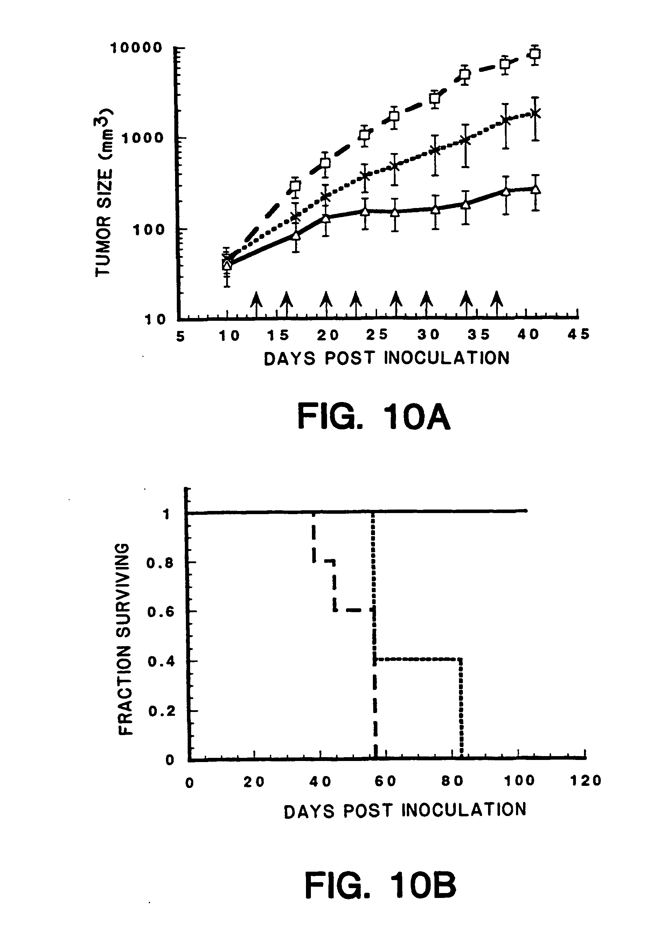

Figures 10A and 10B show *in vivo* tumor suppression and increased survival time with A/M/N/53. H69 (SCLC) tumor cells were injected subcutaneously into nude mice and allowed to develop for 2 weeks. Peritumoral injections of either buffer alone (---), control A/M adenovirus (-x-x-), or A/M/N/53 (-Δ-Δ), both viruses (2 x 10⁹ pfu/injection) were administered twice per week for a total of 8 doses. Tumor dimensions were measured twice per week and tumor volume was estimated as described in Experiment No. II. A) Tumor size is plotted for each virus versus time (days) post inoculation of H69 cells. Error bars indicate the mean tumor size +/- SEM for each group of 5 animals. Arrows indicate days virus injections. B) Mice were monitored for survival and the fraction of mice surviving per group versus time post inoculation of buffer alone (----), control A/M (··· ··· ···) or A/M/N/53 (―) virus treated H69 cells is plotted.

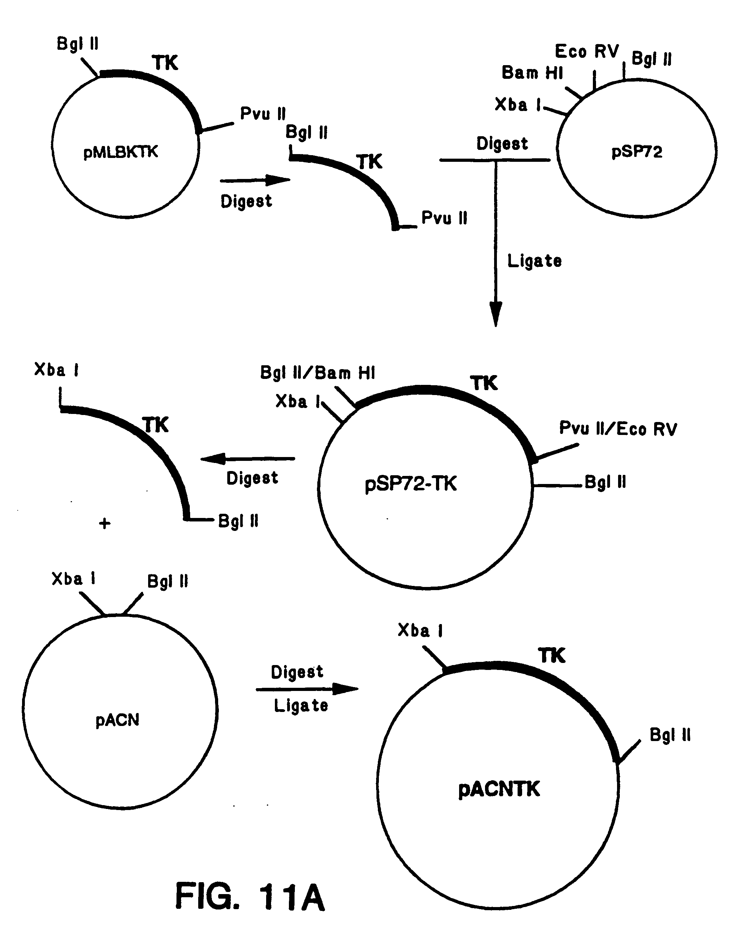

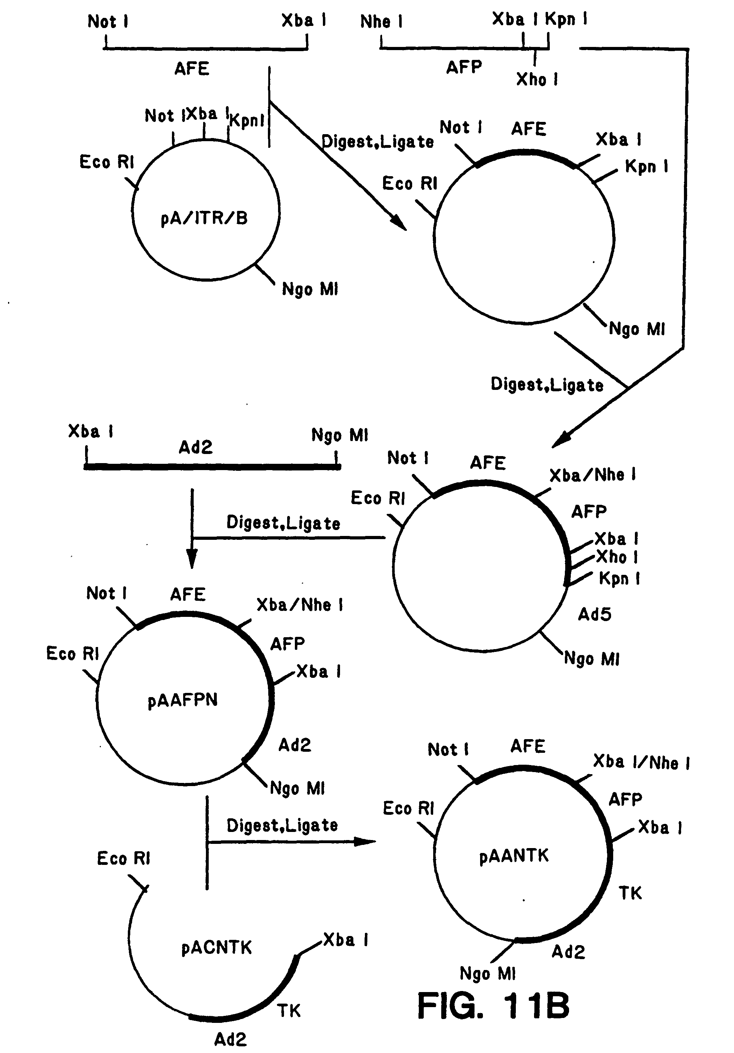

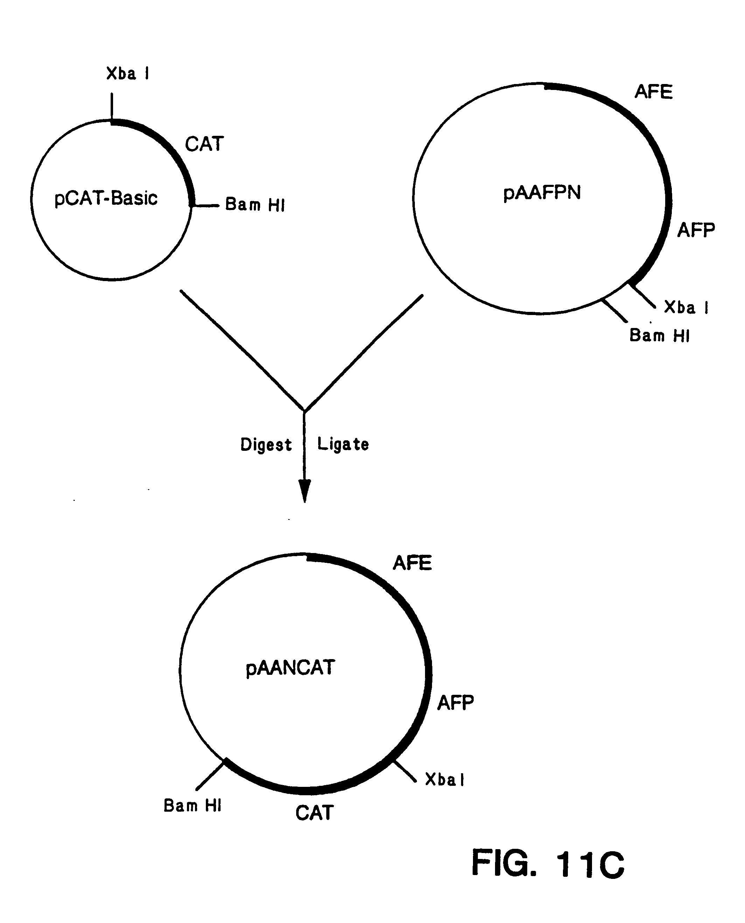

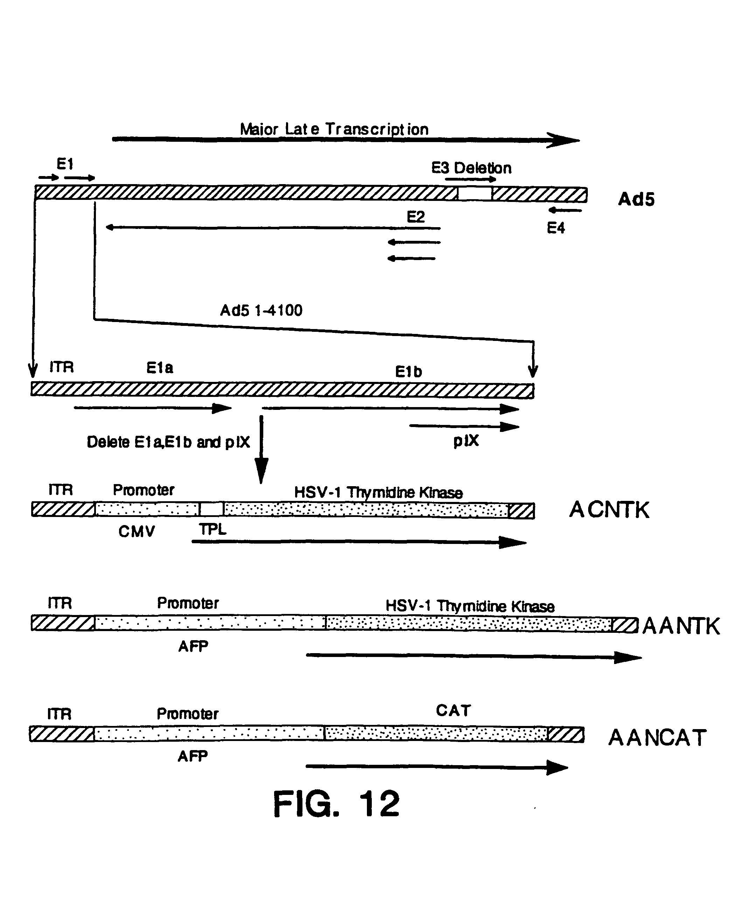

Figures 11A through 11C show maps of recombinant plasmid constructions. Plasmids were constructed as detailed in below. Bold lines in the constructs indicate genes of interest while boldface type indicates the restriction sites used to generate the fragments to be ligated together to form the subsequent plasmid as indicated by the arrows. In Figure 11A, the plasmid pACNTK was constructed by subcloning the HSV-TK gene from pMLBKTK (ATCC No. 39369) into the polylinker of a cloning vector, followed by isolation of the TK gene with the desired ends for cloning into the pACN vector. The pACN vector contains adenoviral sequences necessary for *in vivo* recombination to occur to form recombinant adenovirus (see Figure 12). In Figure 11B, the construction of the plasmid pAANTK is shown beginning with PCR amplified fragments encoding the α-fetoprotein enhancer (AFP-E) and promoter (AFP-P) regions subcloned through several steps into a final plasmid where the AFP enhancer and promoter are upstream of the HSV-TK gene followed by adenovirus Type 2 sequences necessary for *in vivo* recombination to occur to form recombinant adenovirus. In Figure 11C, the construction of the plasmid pAANCAT is shown beginning with the isolation of the chloramphenicol acetyltransferase (CAT) gene from a commercially available plasmid and subcloning it into the pAAN plasmid (see above), generating the final plasmid pAANCAT where the AFP enhancer/promoter direct transcription of the CAT gene in an adenovirus sequence background.
Figure 12 is a schematic map of recombinant adenoviruses ACNTK, AANTK and AANCAT. To construct recombinant adenoviruses from the plasmids described in Figure 11, 4 parts (20 µg) of either plasmid pACNTK, pAANTK, or pAANCAT were linearized with Eco R1 and cotransfected with 1 part (5 µg) of the large fragment of Cla 1 digested recombinant adenovirus (rACβ-gal) containing an E3 region deletion (Wills et al., 1994). In the resulting viruses, the Ad 5 nucleotides 360 - 4021 are replaced by either the CMV promoter and tripartite leader cDNA (TPL) or the α-fetoprotein enhancer and promoter (AFP) driving expression of the HSV-1 TK or CAT gene as indicated. The resulting recombinant adenoviruses are designated ACNTK, AANTK, and AANCAT respectively.

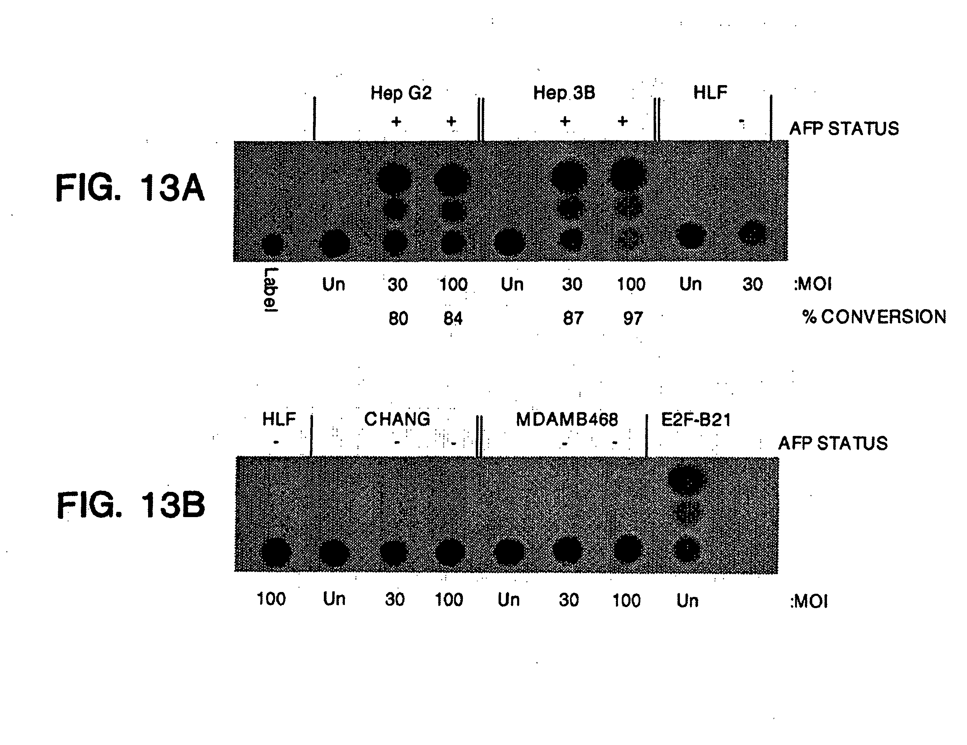

Figure 13 shows promoter specificity of CAT expression in the recombinant adenoviral vectors. Two (2) X 10⁶ of the designated cell lines were infected at MOIs = 30 or 100 of the recombinant adenovirus AANCAT as indicated or left uninfected (UN). Hep G2 and Hep 3B cells express α-fetoprotein whereas the other cell lines do not. After three days, the cells were harvested, extract volumes were adjusted for equal total protein concentrations, and CAT activity was measured as described in Methods section, below. An equal number of uninfected cells served as individual controls for background CAT activity, while ¹⁴C labelled chloramphenicol (¹⁴C-only) and extract from a stable cell line (B21) expressing CAT activity served as negative and positive controls respectively. Percent conversion of acetyl CoA is indicated, demonstrating that CAT expression is limited to those cells expressing α-fetoprotein.

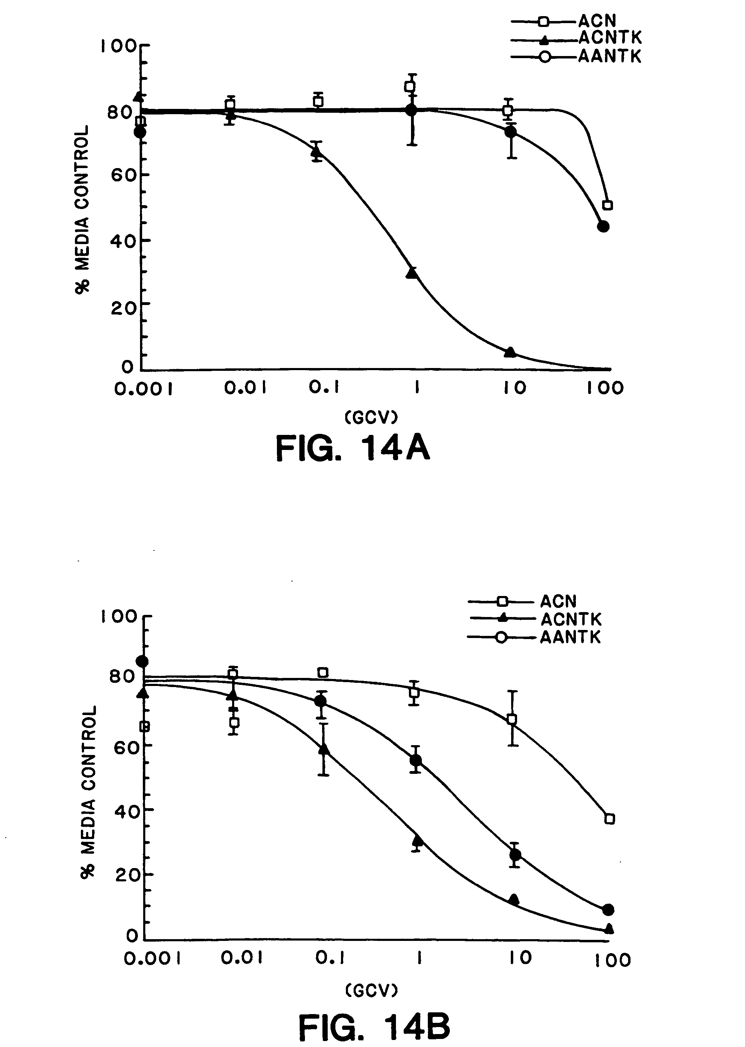

Figure 14 shows the effects of TK/GCV treatment on hepatocellular carcinoma cell lines and the effects of promoter specificity. Hep-G2 (AFP positive) and HLF (AFP negative) cell lines were infected overnight with ACNTK [-Δ-] AANTK [-▲-], or control ACN [-□-] virus at an infection multiplicity of 30 and subsequently treated with a single dose of ganciclovir at the indicated concentrations. Cell proliferation was assessed by adding ³H-thymidine to the cells approximately 18 hours prior to harvest. ³H-thymidine incorporation into cellular nucleic acid was measured 72 hours after infection (Top Count, Packard and expressed as a percent (mean +/- S.D.) of untreated control. The results show a non-selective dose dependent inhibition of proliferation with the CMV driven construct, while AFP driven TK selectively inhibits Hep-G2.

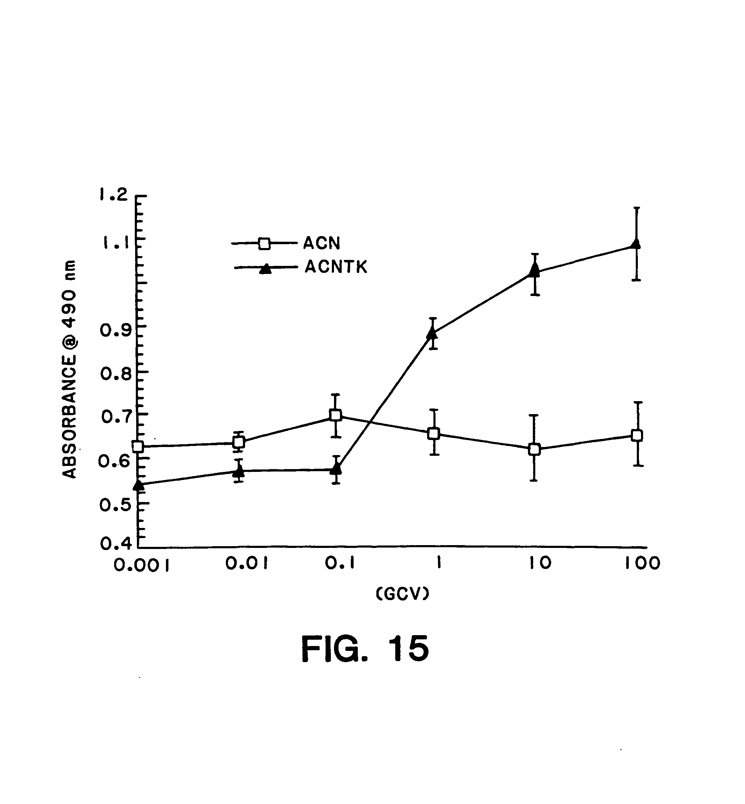

Figure 15 shows cytotoxicity of ACNTK plus ganciclovir in HCC. HLF cells were infected at an MOI of 30 with either ACNTK [-●-] or the control virus ACN [-□-] and treated with ganciclovir at the indicated doses. Seventy-two (72) hours after ganciclovir treatment, the amount of lactate dehydrogenase (LDH) released into the cell supernatant were measured colorimetrically and plotted (mean+/-SEM) versus ganciclovir concentration for the two virus treated groups.

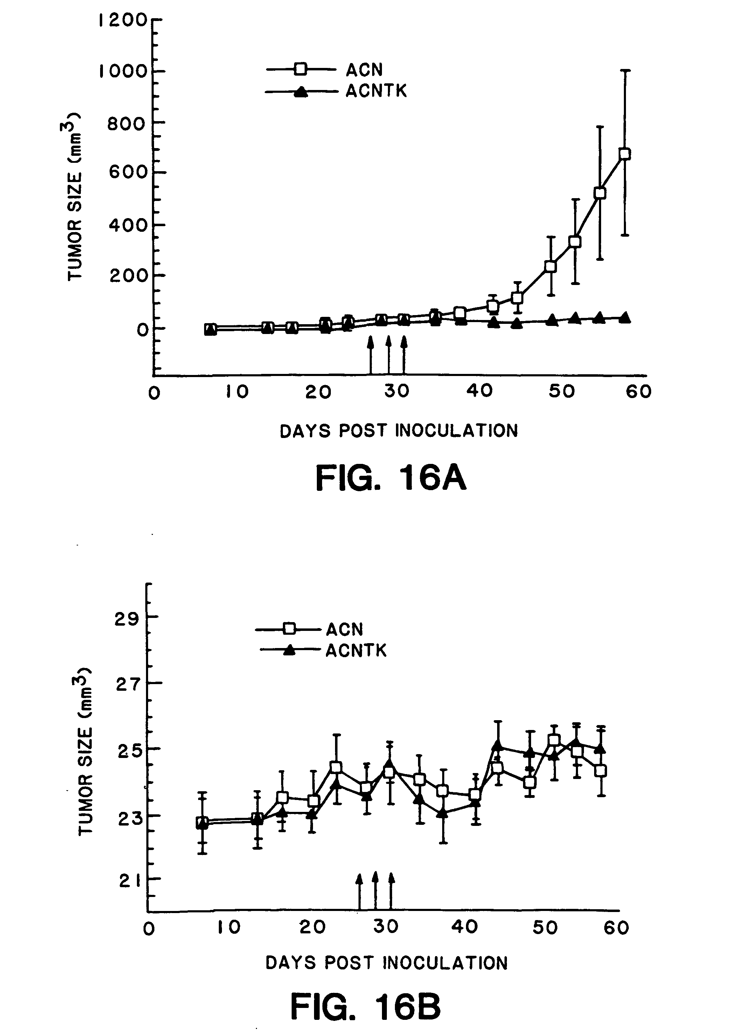

Figures 16A and 16B show the effect of ACNTK plus ganciclovir on established hepatocellular carcinoma (HCC) tumors in nude mice. One (1) X 10⁷ Hep 3B cells were injected subcutaneously into the flank of female nude mice and allowed to grow for 27 days. Mice then received intratumoral and peritumoral injections of either the ACNTK [-●-] or control ACN [-□-] virus (1 X 10⁹ iu in 100 µl volume) every other day for a total of three doses (indicated by arrows). Injections of ganciclovir (100 mg/kg ip) began 24 hours after the initial virus dose and continued for a total of 10 days. In Figure 6A, tumor sizes are plotted for each virus versus days post infection (mean +/- SEM). In Figure 6B, body weight for each virus-treated animal group is plotted as the mean +/-SEM versus days post infection.

### DETAILED DESCRIPTION OF THE INVENTION

To reduce the frequency of contamination with wild-type adenovirus, it is desirable to improve either the virus or the cell line to reduce the probability of recombination. For example, an adenovirus from a group with low homology to the group C viruses could be used to engineer recombinant viruses with little propensity for recombination with the Ad5 sequences in 293 cells. However, an alternative, easier means of reducing the recombination between viral and cellular sequences is to increase the size of the deletion in the recombinant virus and thereby reduce the extent of shared sequence between it and the Ad5 genes in the 293 cells.

Deletions which extend past 3.5 kb from the 5' end of the adenoviral genome affect the gene for adenoviral protein IX and have not been considered desirable in adenoviral vectors (see below).

The protein IX gene of the adenoviruses encodes a minor component of the outer adenoviral capsid which stabilizes the group-of-nine hexons which compose the majority of the viral capsid (Stewart (1993)). Based upon study of adenovirus deletion mutants, protein IX initially was thought to be a non-essential component of the adenovirus, although its absence was associated with greater heat lability than observed with wild-type virus (Colby and Shenk (1981)). More recently it was discovered that protein IX is essential for packaging full length viral DNA into capsids and that in the absence of protein IX, only genomes at least 1 kb smaller than wild-type could be propagated as recombinant viruses (Ghosh-Choudhury *et al*. (1987)). Given this packaging limitation, protein IX deletions deliberately have not been considered in the design of adenoviral vectors.
For example, the adenoviral vector disclosed in Venkatesh L.K. et al. (Proc. Natl. Acad. Sci. USA (1990) Vol. 87, pp. 8746-8750) contains a deletion in the E1a/E1b region that extends from a PvuII restriction site to a BglII restriction site, so that the protein IX encoding gene remains intact.

In this application, reference is made to standard textbooks of molecular biology that contain definitions, methods and means for carrying out basic techniques, encompassed by the present invention. See for example, Sambrook et al. (1989) and the various references cited therein.

Contrary to what has been known in the art, this invention claims the use of recombinant adenoviruses bearing deletions of the protein IX gene as a means of reducing the risk of wild-type adenovirus contamination in virus preparations for use in diagnostic and therapeutic applications such as gene therapy. As used herein, the term "recombinant" is intended to mean a progeny formed as the result of genetic engineering. These deletions can remove an additional 500 to 700 base pairs of DNA sequence that is present in conventional E1 deleted viruses and is available for recombination with the Ad5 sequences integrated in 293 cells. Recombinant adenoviruses based on any group C virus, serotype 1, 2, 5 and 6, are included in this invention. Also encompassed by this invention is a hybrid Ad2/Ad5 based recombinant virus expressing the human p53 cDNA from the adenovirus type 2 major late promoter. This construct was assembled as shown in Figure 1. The resultant virus bears a 5' deletion of adenoviral sequences extending from about nucleotide 357 to 4020 and eliminates the E1a and E1b genes as well as the entire protein IX coding sequence, leaving the polyadenylation site shared by the E1b and protein IX genes intact for use in terminating transcription of any desired gene. A separate embodiment is shown in Figure 4. Alternatively, the deletion can be extended an additional 30 to 40 base pairs without affecting the adjacent gene for protein IVa2, although in that case an exogenous polyadenylation signal is provided to terminate transcription of genes inserted into the recombinant virus. The initial virus constructed with this deletion is easily propagated in 293 cells with no evidence of wild-type viral contamination and directs robust p53 expression from the transcriptional unit inserted at the site of the deletion.

The insert capacity of recombinant viruses bearing the protein IX deletion described above is approximately 2.6 kb. This is sufficient for many genes including the p53 cDNA. Insert capacity can be increased by introducing other deletions into the adenoviral backbone, for example, deletions within early regions 3 or 4 (for review see: Graham and Prevec (1991)). For example, the use of an adenoviral backbone containing a 1.9 kb deletion of non-essential sequence within early region 3. With this additional deletion, the insert capacity of the vector is increased to approximately 4.5 kb, large enough for many larger cDNAs, including that of the retinoblastoma tumor suppressor gene.

A recombinant adenovirus expression vector characterized by a total deletion of the adenoviral protein IX DNA and having a gene encoding a foreign protein, or a functional fragment or mutant thereof is provided by this invention. These vectors are useful for the safe recombinant production of diagnostic and therapeutic polypeptides and proteins, and more importantly, for the introduction of genes in gene therapy. Thus, for example, the adenoviral vector of this invention can contain a foreign gene for the expression of a protein effective in regulating the cell cycle, such as p53, Rb, or mitosin, or in inducing cell death, such as the conditional suicide gene thymidine kinase. (The latter must be used in conjunction with a thymidine kinase metabolite in order to be effective).Any expression cassette can be used in the vectors of this invention. An "expression cassette" means a DNA molecule having a transcription promoter/enhancer such as the CMV promotor enhancer, etc., a foreign gene, and in some embodiments defined below, a polyadentlyation signal. As used herein, the term "foreign gene" is intended to mean a DNA molecule not present in the exact orientation and position as the counterpart DNA molecule found in wild-type adenovirus. The foreign gene is a DNA molecule up to 4.5 kilobases. "Expression vector" means a vector that results in the expression of inserted DNA sequences when propagated in a suitable host cell, i.e., the protein or polypeptide coded for by the DNA is synthesized by the host's system.

Inducible promoters also can be used in the adenoviral vector of this invention. These promoters will initiate transcription only in the presence of an additional molecule. Examples of inducible promoters include those obtainable from a β-interferon gene, a heat shock gene, a metallothionine gene or those obtainable from steroid hormone-responsive genes. Tissue specific expression has been well characterized in the field of gene expression and tissue specific and inducible promoters such as these are very well known in the art. These genes are used to regulate the expression of the foreign gene after it has been introduced into the target cell.

Also provided by this invention is a recombinant adenovirus expression vector, as described above, having less extensive deletions of the protein IX gene sequence extending from 3500 bp from the 5' viral termini to approximately 4000 bp, in one embodiment. In a separate embodiment, the recombinant adenovirus expression vector can have a further deletion of a non-essential DNA sequence in adenovirus early region 3 and/or 4 and/or deletion of the DNA sequences designated adenovirus E1a and E1b. In this embodiment, foreign gene is a DNA molecule of a size up to 4.5 kilobases.

A further embodiment has a deletion of up to forty nucleotides positioned 3' to the E1a and E1b deletion and pIX and a foreign DNA molecule encoding a polyadenylation signal inserted into the recombinant vector in a position relative to the foreign gene to regulate the expression of the foreign gene.

For the purposes of this invention, the recombinant adenovirus expression vector can be derived from wild-type group adenovirus, serotype 1, 2, 5 or 6.

In one embodiment, the recombinant adenovirus expression vector has a foreign gene coding for a functional tumor suppressor protein, or a biologically active fragment thereof. As used herein, the term "functional" as it relates to a tumor suppressor gene, refers to tumor suppressor genes that encode tumor suppressor proteins that effectively inhibit a cell from behaving as a tumor cell. Functional genes can include, for instance, wild type of normal genes and modifications of normal genes that retains its ability to encode effective tumor suppressor proteins and other anti-tumor genes such as a conditional suicide protein or a toxin.

Similarly, "non-functional" as used herein is synonymous with "inactivated." Non-functional or defective genes can be caused by a variety of events, including for example point mutations, deletions, methylation and others known to those skilled in the art.

As used herein, an "active fragment" of a gene includes smaller portions of the gene that retain the ability to encode proteins having tumor suppressing activity. p56^{RB}, described more fully below, is but one example of an active fragment of a functional tumor suppressor gene. Modifications of tumor suppressor genes are also contemplated within the meaning of an active fragment, such as additions, deletions or substitutions, as long as the functional activity of the unmodified gene is retained.

Another example of a tumor suppressor gene is retinoblastoma (RB). The complete RB cDNA nucleotide sequences and predicted amino acid sequences of the resulting RB protein (designated p110^{RB}) are shown in Lee *et al*. (1987) and in Figure 3. Also useful to express retinoblastoma tumor suppressor protein is a DNA molecule encoding the amino acid sequence shown in Figure 2 or having the DNA sequence shown in Figure 3. A truncated version of p110^{RB}, called p56^{RB} also is useful. For the sequence of p56^{RB}, see Huang *et al*. (1991). Additional tumor suppressor genes can be used in the vectors of this invention. For illustration purposes only, these can be p16 protein (Kamb *et al*. (1994)), p21 protein, Wilm's tumor WT1 protein, mitosin, h-NUC, or colon carcinoma DCC protein. Mitosin is described in X. Zhu and W-H Lee, U.S. Application Serial No. 08/141,239, filed October 22, 1993, and a subsequent continuation-in-part by the same inventors, attorney docket number P-CJ 1191, filed October 24, 1994. Similarly, h-NUC is described by W-H Lee and P-L Chen, U.S. Application Serial No. 08/170,586, filed December 20, 1993.

As is known to those of skill in the art, the term "protein" means a linear polymer of amino acids joined in a specific sequence by peptide bonds. As used herein, the term "amino acid" refers to either the D or L stereoisomer form of the amino acid, unless otherwise specifically designated. Also encompassed are equivalent proteins or equivalent peptides, e.g., having the biological activity of purified wild type tumor suppressor protein. "Equivalent proteins" and "equivalent polypeptides" refer to compounds that depart from the linear sequence of the naturally occurring proteins or polypeptides, but which have amino acid substitutions that do not change its biologically activity. These equivalents can differ from the native sequences by the replacement of one or more amino acids with related amino acids, for example, similarly charged amino acids, or the substitution or modification of side chains or functional groups.

Also encompassed within the definition of a functional tumor suppressor protein is any protein whose presence reduces the tumorigenicity, malignancy or hyperproliferative phenotype of the host cell. Examples of tumor suppressor proteins within this definition include, but are not limited to p110^{RB}, p56^{RB}, mitosin, h-NUC and p53. "Tumorigenicity" is intended to mean having the ability to form tumors or capable of causing tumor formation and is synonymous with neoplastic growth. "Malignancy" is intended to describe a tumorigenic cell having the ability to metastasize and endanger the life of the host organism. "Hyperproliferative phenotype" is intended to describe a cell growing and dividing at a rate beyond the normal limitations of growth for that cell type. "Neoplastic" also is intended to include cells lacking endogenous functional tumor suppressor protein or the inability of the cell to express endogenous nucleic acid encoding a functional tumor suppressor protein.

An example of a vector of this invention is a recombinant adenovirus expression vector having a foreign gene coding for p53 protein or an active fragment thereof is provided by this invention. The coding sequence of the p53 gene is set forth below in Table I.

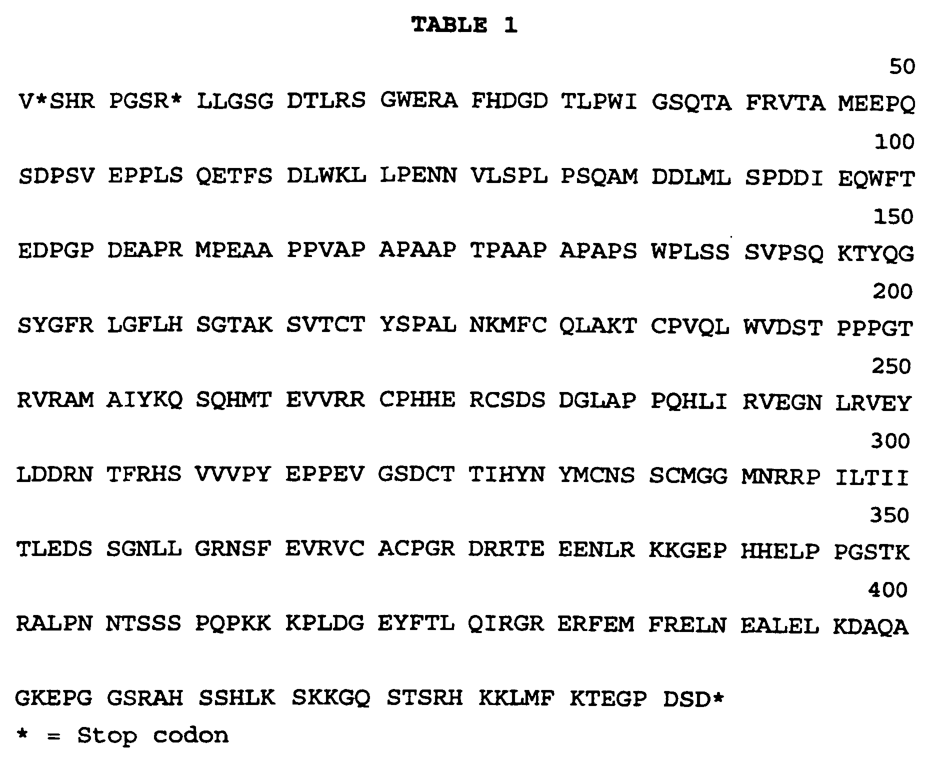

Any of the expression vectors described herein are useful as compositions for diagnosis or therapy. The vectors can be used for screening which of many tumor suppressor genes would be useful in gene therapy. For example, a sample of cells suspected of being neoplastic can be removed from a subject and mammal. The cells can then be contacted, under suitable conditions and with an effective amount of a recombinant vector of this invention having inserted therein a foreign gene encoding one of several functional tumor suppressor genes. Whether the introduction of this gene will reverse the malignant phenotype can be measured by colony formation in soft agar or tumor formation in nude mice. If the malignant phenotype is reversed, then that foreign gene is determined to be a positive candidate for successful gene therapy for the subject or mammal. When used pharmaceutically, they can be combined with one or more pharmaceutically acceptable carriers. Pharmaceutically acceptable carriers are well known in the art and include aqueous solutions such as physiologically buffered saline or other solvents or vehicles such as glycols, glycerol, vegetable oils (eg., olive oil) or injectable organic esters. A pharmaceutically acceptable carrier can be used to administer the instant compositions to a cell *in vitro* or to a subject *in vivo*.

A pharmaceutically acceptable carrier can contain a physiologically acceptable compound that acts, for example, to stabilize the composition or to increase or decrease the absorption of the agent. A physiologically acceptable compound can include, for example, carbohydrates, such as glucose, sucrose or dextrans, antioxidants, such as ascorbic acid or glutathione, chelating agents, low molecular weight proteins or other stabilizers or excipients. Other physiologically acceptable compounds include wetting agents, emulsifying agents, dispersing agents or preservatives, which are particularly useful for preventing the growth or action of microorganisms. Various preservatives are well known and include, for example, phenol and ascorbic acid. One skilled in the art would know that the choice of a pharmaceutically acceptable carrier, including a physiologically acceptable compound, depends, for example, on the route of administration of the polypeptide and on the particular physio-chemical characteristics of the specific polypeptide. For example, a physiologically acceptable compound such as aluminum monosterate or gelatin is particularly useful as a delaying agent, which prolongs the rate of absorption of a pharmaceutical composition administered to a subject. Further examples of carriers, stabilizers or adjutants can be found in Martin, Remington's Pharm. Sci., 15th Ed. (Mack Publ. Co., Easton, 1975). The pharmaceutical composition also can be incorporated, if desired, into liposomes, microspheres or other polymer matrices (Gregoriadis, Liposome Technology, Vol. 1 (CRC Press, Boca Raton, Florida 1984)). Liposomes, for example, which consist of phospholipids or other lipids, are nontoxic, physiologically acceptable and metabolizable carriers that are relatively simple to make and administer.

As used herein, "pharmaceutical composition" refers to any of the compositions of matte described herein in combination with one or more of the above pharmaceutically acceptable carriers. The compositions can then be administered therapeutically or prophylactically. They can be contacted with the host cell *in vivo, ex vivo,* or *in vitro,* in an effective amount. *In vitro* and *ex vivo* means of contacting host cells are provided below. When practiced *in vivo*, methods of administering a pharmaceutical containing the vector of this invention, are well known in the art and include but are not limited to, administration orally, intra-tumorally, intravenously, intramuscularly or intraperitoneal. Administration can be effected continuously or intermittently and will vary with the subject and the condition to be treated, e.g., as is the case with other therapeutic compositions (Landmann *et al*. (1992); Aulitzky *et al*. (1991); Lantz *et al*. (1990); Supersaxo *et al*. (1988); Demetri *et al*. (1989); and LeMaistre *et al*. (1991)).

Further provided by this invention is a transformed procaryotic or eucaryotic host cell, for example an animal cell or mammalian cell, having inserted a recombinant adenovirus expression vector described above. Suitable procaryotic cells include but are not limited to bacterial cells such as *E. coli* cells. Methods of transforming host cells with retroviral vectors are known in the art, see Sambrook et al. (1989) and include, but are not limited to transfection, electroporation, and microinjection.

As used throughout this application, the term animal is intended to be synonymous with mammal and is to include, but not be limited to bovine, porcine, feline, simian, canine, equine, murine, rat or human. Additional host cells include but are not limited to any neoplastic or tumor cell, such as osteosarcoma, ovarian carcinoma, breast carcinoma, melanoma, hepatocarcinoma, lung cancer, brain cancer, colorectal cancer, hematopoietic cell, prostate cancer, cervical carcinoma, retinoblastoma, esophageal carcinoma, bladder cancer, neuroblastoma, or renal cancer.

Additionally, any eucaryotic cell line capable of expressing E1a and E1b or E1a, E1b and pIX is a suitable host for this vector. In one embodiment, a suitable eucaryotic host cell is the 293 cell line available from the American Type Culture Collection, 12301 Parklawn Drive, Rockville, Maryland, U.S.A. 20231.

Any of the transformed host cells described herein are useful as compositions for diagnosis or therapy. When used pharmaceutically, they can be combined with various pharmaceutically acceptable carriers. Suitable pharmaceutically acceptable carriers are well known to those of skill in the art and, for example, are described above. The compositions can then be administered therapeutically or prophylactically, in effective amounts, described in more detail below.

A method of transforming a host cell also is provided. This method provides contacting a host cell, i.e., a procaryotic or eucaryotic host cell, with any of the expression vectors described herein and under suitable conditions. The contacting can be effected *in vitro, in vivo, or ex vivo,* using methods well known in the art (Sambrook et al. (1989)) and using effective amounts of the expression vectors. Also provided in this invention is a method of producing a recombinant protein or polypeptide by growing the transformed host cell under suitable conditions favoring the transcription and translation of the inserted foreign gene. Methods of recombinant expression in a variety of host cells, such as mammalian, yeast, insect or bacterial cells, are widely known, including those described in Sambrook et al., supra. The translated foreign gene can then be isolated by convention means, such as column purification or purification using an anti-protein antibody. The isolated protein or polypeptide also is intended within the scope of this invention. As used herein, purified or isolated mean substantially free of native proteins or nucleic acids normally associated with the protein or polypeptide in the native or host cell environment.

Also provided by this invention are non-human animals having inserted therein the expression vectors or transformed host cells of this invention. These "transgenic" animals are made using methods well known to those of skill in the art, for example as described in U.S. Patent No. 5,175,384 or by conventional *ex vivo* therapy techniques, as described in Culver et al. (1991).

As shown in detail below, the recombinant adenoviruses expressing a tumor suppressor wild-type p53, as described above, can efficiently inhibit DNA synthesis and suppress the growth of a broad range of human tumor cell types, including clinical targets. Furthermore, recombinant adenoviruses can express tumor suppression genes such as p53 in an *in vivo* established tumor without relying on direct injection into the tumor or prior *ex vivo* treatment of the cancer cells. The p53 expressed is functional and effectively suppresses tumor growth *in vivo* and significantly increases survival time in a nude mouse model of human lung cancer.

Thus, the vectors of this invention are particularly suited for gene therapy. Accordingly, methods of gene therapy utilizing these vectors are within the scope of this invention. The vector is purified and then an effective amount is administered *in vivo* or *ex vivo* into the subject. Methods of gene therapy are well known in the art, see, for example, Larrick, J.W. and Burck, K.L. (1991) and Kreigler, M. (1990). "Subject" means any animal, mammal, rat, murine, bovine, porcine, equine, canine, feline or human patient. When the foreign gene codes for a tumor suppressor gene or other anti-tumor protein, the vector is useful to treat or reduce hyperproliferative cells in a subject, to inhibit tumor proliferation in a subject or to ameliorate a particular related pathology. Pathologic hyperproliferative cells are characteristic of the following disease states, thyroid hyperplasia - Grave's Disease, psoriasis, benign prostatic hypertrophy, Li-Fraumeni syndrome including breast cancer, sarcomas and other neoplasms, bladder cancer, colon cancer, lung cancer, various leukemias and lymphomas. Examples of non-pathologic hyperproliferative cells are found, for instance, in mammary ductal epithelial cells during development of lactation and also in cells associated with wound repair. Pathologic hyperproliferative cells characteristically exhibit loss of contact inhibition and a decline in their ability to selectively adhere which implies a change in the surface properties of the cell and a further breakdown in intercellular communication. These changes include stimulation to divide and the ability to secrete proteolytic enzymes.

Moreover, the present invention relates to a method for depleting a suitable sample of pathologic mammalian hyperproliferative cells contaminating hematopoietic precursors during bone marrow reconstitution via the introduction of a wild type tumor suppressor gene into the cell preparation using the vector of this invention (whether derived from autologous peripheral blood or bone marrow). As used herein, a "suitable sample" is defined as a heterogeneous cell preparation obtained from a patient, e.g., a mixed population of cells containing both phenotypically normal and pathogenic cells. "Administer" includes, but is not limited to introducing into the cell or subject intravenously, by direct injection into the tumor, by intra-tumoral injection, by intraperitoneal administration, by aerosol administration to the lung or topically. Such administration can be combined with a pharmaceutically-accepted carrier, described above.

The term "reduced tumorigenicity" is intended to mean tumor cells that have been converted into less tumorigenic or non-tumorigenic cells. Cells with reduced tumorigenicity either form no tumors *in vivo* or have an extended lag time of weeks to months before the appearance of *in vivo* tumor growth and/or slower growing three dimensional tumor mass compared to tumors having fully inactivated or non-functional tumor suppressor gene.

As used herein, the term "effective amount" is intended to mean the amount of vector or anti-cancer protein which achieves a positive outcome on controlling cell proliferation. For example, one dose contains from about 10⁸ to about 10¹³ infectious units. A typical course of treatment would be one such dose a day over a period of five days. An effective amount will vary on the pathology or condition to be treated, by the patient and his status, and other factors well known to those of skill in the art. Effective amounts are easily determined by those of skill in the art.

Also within the scope of this invention is a method of ameliorating a pathology characterized by hyperproliferative cells or genetic defect in a subject by administering to the subject an effective amount of a vector described above containing a foreign gene encoding a gene product having the ability to ameliorate the pathology, under suitable conditions. As used herein, the term "genetic defect" means any disease or abnormality that results from inherited factors, such as sickle cell anemia or Tay-Sachs disease.

This invention also provides a method for reducing the proliferation of tumor cells in a subject by introducing into the tumor mass an effective amount of an adenoviral expression vector containing an anti-tumor gene other than a tumor suppressor gene. The anti-tumor gene can encode, for example, thymidine kinase (TK). The subject is then administered an effective amount of a therapeutic agent, which in the presence of the anti-tumor gene is toxic to the cell. In the specific case of thymidine kinase, the therapeutic agent is a thymidine kinase metabolite such as ganciclovir (GCV), 6-methoxypurine arabinonucleoside (araM), or a functional equivalent thereof. Both the thymidine kinase gene and the thymidine kinase metabolite must be used concurrently to be toxic to the host cell. However, in its presence, GCV is phosphorylated and becomes a potent inhibitor of DNA synthesis whereas araM gets converted to the cytotoxic anabolite araATP. Other anti-tumor genes can be used as well in combination with the corresponding therapeutic agent to reduce the proliferation of tumor cells. Such other gene and therapeutic agent combinations are known by one skilled in the art. Another example would be the vector of this invention expressing the enzyme cytosine deaminase. Such vector would be used in conjunction with administration of the drug 5-fluorouracil (Austin and Huber, 1993), or the recently described E. Coli Deo Δ gene in combination with 6-methyl-purine-2'-deosribonucleoside (Sorscher et al 1994).

As with the use of the tumor suppressor genes described previously, the use of other anti-tumor genes, either alone or in combination with the appropriate therapeutic agent provides a treatment for the uncontrolled cell growth or proliferation characteristic of tumors and malignancies. Thus, this invention provides a therapy to stop the uncontrolled cellular growth in the patient thereby alleviating the symptoms of the disease or cachexia present in the patient. The effect of this treatment includes, but is not limited to, prolonged survival time of the patient, reduction in tumor mass or burden, apoptosis of tumor cells or the reduction of the number of circulating tumor cells. Means of quantifying the beneficial effects of this therapy are well known to those of skill in the art.

The invention provides a recombinant adenovirus expression vector characterized by the total deletion of the adenoviral protein IX DNA and having a foreign gene encoding a foreign protein, wherein the foreign protein is a suicide gene or functional equivalent thereof. The anti-cancer gene TK, described above, is an example of a suicide gene because when expressed, the gene product is, or can be made to be lethal to the cell. For TK, lethality is induced in the presence of GCV. The TK gene is derived from herpes simplex virus by methods well known to those of skill in the art. The plasmid pMLBKTK in E. coli HB101 (from ATCC #39369) is a source of the herpes simplex virus (HSV-1) thymidine kinase (TK) gene for use in this invention. However, many other sources exist as well.

The TK gene can be introduced into the tumor mass by combining the adenoviral expression vector with a suitable pharmaceutically acceptable carrier. Introduction can be accomplished by, for example, direct injection of the recombinant adenovirus into the tumor mass. For the specific case of a cancer such as hepatocellular carcinoma (HCC), direct injection into the hepatic artery can be used for delivery because most HCCs derive their circulation from this artery. To control proliferation of the tumor, cell death is induced by treating the patients with a TK metabolite such as ganciclovir to achieve reduction of tumor mass. The TK metabolite can be administered, for example, systemically, by local innoculation into the tumor or in the specific case of HCC, by injection into the hepatic artery. The TK metabolite is preferably administered at least once daily but can be increased or decreased according to the need. The TK metabolite can be administered simultaneous or subsequent to the administration of the TK containing vector. Those skilled in the art know or can determine the dose and duration which is therapeutically effective.

A method of tumor-specific delivery of a tumor suppressor gene is accomplished by contacting target tissue in an animal with an effective amount of the recombinant adenoviral expression vector of this invention. The gene is intended to code for an anti-tumor agent, such as a functional tumor suppressor gene or suicide gene. "Contacting" is intended to encompass any delivery method for the efficient transfer of the vector, such as intra-tumoral injection.

The use of the adenoviral vector of this invention to prepare medicaments for the treatment of a disease or for therapy is further provided by this invention.

### EXPERIMENT NO. I

Plasmid pAd/MLP/p53/E1b- was used as the starting material for these manipulations. This plasmid is based on the pBR322 derivative pML2 (pBR322 deleted for base pairs 1140 to 2490) and contains adenovirus type 5 sequences extending from base pair 1 to base pair 5788 except that it is deleted for adenovirus type 5 base pairs 357 to 3327. At the site of the Ad5 357/3327 deletion a transcriptional unit is inserted which is comprised of the adenovirus type 2 major late promoter, the adenovirus type 2 tripartite leader cDNA and the human p53 cDNA. It is a typical E1 replacement vector deleted for the Ad5 E1a and E1b genes but containing the Ad5 protein IX gene (for review of Adenovirus vectors see: Graham and Prevec (1992)). Ad2 DNA was obtained from Gibco BRL. Restriction endonucleases and T4 DNA ligase were obtained from New England Biolabs. *E. coli* DH5α competent cells were purchased from Gibco BRL and 293 cells were obtained from the American Type Culture Collection (ATCC). Prep-A-Gene DNA purification resin was obtained from BioRad. LB broth bacterial growth medium was obtained from Difco. Qiagen DNA purification columns were obtained from Qiagen, Inc. Ad5 dl327 was obtained from R.J. Schneider, NYU. The MBS DNA transfection kit was purchased from Stratagene.

One (1) µg pAd/MLP/p53/E1b- was digested with 20 units each of restriction enzymes Ecl 136II and NgoMI according to the manufacturer's recommendations. Five (5) µg Ad2 DNA was digested with 20 units each of restriction endonucleases DraI and NgoMI according to the manufacturer's recommendations. The restriction digestions were loaded into separate lanes of a 0.8% agarose gel and electrophoresed at 100 volts for 2 hours. The 4268 bp restriction fragment from the Pad/MLP/p53/E1b- sample and the 6437 bp fragment from the Ad2 sample were isolated from the gel using Prep-A-Gene DNA extraction resin according to the manufacturer's specifications. The restriction fragments were mixed and treated with T4 DNA ligase in a total volume of 50 µl at 16°C for 16 hours according to the manufacturer's recommendations. Following ligation 5 µl of the reaction was used to transform *E. coli* DH5α cells to ampicillin resistance following the manufacturer's procedure. Six bacterial colonies resulting from this procedure were used to inoculate separate 2 ml cultures of LB growth medium and incubated overnight at 37°C with shaking. DNA was prepared from each bacterial culture using standard procedures (Sambrook *et al*. (1989)). One fourth of the plasmid DNA from each isolate was digested with 20 units of restriction endonuclease XhoI to screen for the correct recombinant containing XhoI restriction fragments of 3627, 3167, 2466 and 1445 base pairs. Five of six screened isolates contained the correct plasmid. One of these was then used to inoculate a 1 liter culture of LB medium for isolation of large quantities of plasmid DNA. Following overnight incubation plasmid DNA was isolated from the 1 liter culture using Qiagen DNA purification columns according to the manufacturer's recommendations. The resulting plasmid was designated Pad/MLP/p53/PIX-. Samples of this plasmid were deposited with the American Type Culture Collection, 12301 Parklawn Drive, Rockville, Maryland, U.S.A., 12301, on October 22, 1993. The deposit was made under the provisions of the Budapest Treaty on the International Deposit of Microorganisms for the Purpose of Patent Procedure. The deposit was accorded ATCC Accession No. 75576.

To construct a recombinant adenovirus, 10 µg Pad/MLP/p53/PIX- were treated with 40 units of restriction endonuclease EcoRI to linearize the plasmid. Adenovirus type 5 dl327 DNA (Thimmappaya (1982)) was digested with restriction endonuclease ClaI and the large fragment (approximately 33 kilobase pairs) was purified by sucrose gradient centrifugation. Ten (10) µg of EcoRI treated p53 (Table I) was isolated from pGEM1-p53-B-T (kindly supplied by Dr. Wen Hwa Lee) and inserted into the multiple cloning site of the expression vector pSP72 (Promega) using standard cloning procedures (Sambrook *et al*. (1989)). The p53 insert was recovered from this vector following digestion with XhoI-BglII and gel electrophoresis. The p53 coding sequence was then inserted into either pNL3C or pNL3CMV adenovirus gene transfer vectors (kindly provided by Dr. Robert Schneider) which contain the Ad5 5' inverted terminal repeat and viral packaging signals and the E1a enhancer upstream of either the Ad2 major late promoter (MLP) or the human cytomegalovirus immediate early gene promoter (CMV), followed by the tripartite leader CDNA and Ad 5 sequence 3325-5525 bp in a PML2 background. These new constructs replace the E1 region (bp 360-3325) of Ad5 with p53 driven by either the Ad2 MLP (A/M/53) or the human CMV promoter (A/C/53), both followed by the tripartite leader CDNA (see Figure 4). The p53 inserts use the remaining downstream E1b polyadenylation site. Additional MLP and CMV driven p53 recombinants (A/M/N/53, A/C/N/53) were generated which had a further 705 nucleotide deletion of Ad 5 sequence to remove the protein IX (PIX) coding region. As a control, a recombinant adenovirus was generated from the parental PNL3C plasmid without a p53 insert (A/M). A second control consisted of a recombinant adenovirus encoding the beta-galactosidase gene under the control of the CMV promoter (A/C/β-gal). The plasmids were linearized with either Nru I or Eco RI and co-transfected with the large fragment of a Cla I digested Ad 5 d1309 or d1327 mutants (Jones and Shenk (1979)) using a Ca/PO₄ transfection kit (Stratagene). Viral plaques were isolated and recombinants identified by both restriction digest analysis and PCR using recombinant specific primers against the tripartite leader CDNA sequence with downstream p53 CDNA sequence. Recombinant virus was further purified by limiting dilution, and virus particles were purified and titered by standard methods (Graham and van der Erb (1973); Graham and Prevec (1991)).

### p53 Protein Detection

Saos-2 or Hep 3B cells (5 x 10⁵) were infected with the indicated recombinant adenoviruses for a period of 24 hours at increasing multiplicities of infection (MOI) of plaque forming units of virus/cell. Cells were then washed once with PBS and harvested in lysis buffer (50mM Tris-Hcl Ph 7.5, 250 Mm NaCl, 0.1% NP40, 50mM NaF, 5mM EDTA, 10ug/ml aprotinin, 10 ug/ml leupeptin, and 1mM PMSF). Cellular proteins (approximately 30 µg) were separated by 10% SDS-PAGE and transferred to nitrocellulose. Membranes were incubated with α-p53 antibody PAb 1801 (Novocastro) followed by sheep anti-mouse IgG conjugated with horseradish peroxidase. p53 protein was visualized by chemiluminescence (ECL kit, Amersham) on Kodak XAR-5 film.

### Measurement of DNA Synthesis Rate

Cells (5 x 10³/well) were plated in 96-well titer plates (Costar) and allowed to attach overnight (37°C, 7% CO₂). Cells were then infected for 24 hours with purified recombinant virus particles at MOIs ranging from 0.3 to 100 as indicated. Media were changed 24 hours after infection, and incubation was continued for a total of 72 hours. ³H-thymidine (Amersham, 1µCi/well) was added 18 hours prior to harvest. Cells were harvested on glass fiber filters and levels of incorporated radioactivity were measured in a beta scintillation counter. ³H-thymidine incorporation was expressed as the mean % (+/- SD) of media control and plotted versus the MOI.

### Tumorigenicity in Nude Mice

Approximately 2.4 x 10⁸ Saos-2 cells, plated in T225 flasks, were treated with suspension buffer (1% sucrose in PBS) containing either A/M/N/53 or A/M purified virus at an MOI of 3 or 30. Following an overnight infection, cells were injected subcutaneously into the left and right flanks of BALB/c athymic nude mice (4 mice per group). One flank was injected with the A/M/N/53 treated cells, while the contralateral flank was injected with the control A/M treated cells, each mouse serving as its own control. Animals receiving bilateral injection of buffer treated cells served as additional controls. Tumor dimensions (length, width and height) and body weights were then measured twice per week over an 8 week period. Tumor volumes were estimated for each animal assuming a spherical geometry with radius equal to one-half the average of the measured tumor dimensions.

### Intra-tumoral RNA Analysis

BALB/c athymic nude mice (approximately 5 weeks of age) were injected subcutaneously with 1 x 10⁷ H69 small cell lung carcinoma (SCLC) cells in their right flanks. Tumors were allowed to progress for 32 days until they were approximately 25-50 mm³. Mice received peritumoral injections of either A/C/53 or A/C/β-gal recombinant adenovirus (2 x 10⁹ plaque forming units (pfu)) into the subcutaneous space beneath the tumor mass. Tumors were excised from the animals 2 and 7 days post adenovirus treatment and rinsed with PBS. Tumor samples were homogenized, and total RNA was isolated using a TriReagent kit (Molecular Research Center, Inc.). PolyA RNA was isolated using the PolyATract mRNA Isolation System (Promega), and approximately 10 ng of sample was used for RT-PCR determination of recombinant p53 MRNA expression (Wang *et al.* (1989)). Primers were designed to amplify sequence between the adenovirus tripartite leader CDNA and the downstream p53 CDNA, ensuring that only recombinant, and not endogenous p53 would be amplified.

### p53 Gene Therapy of Established Tumors in Nude Mice

Approximately 1 x 10⁷ H69 (SCLC) tumor cells in 200µl volumes were injected subcutaneously into female BALB/c athymic nude mice. Tumors were allowed to develop for 2 weeks, at which point animals were randomized by tumor size (N=5/group). Peritumoral injections of either A/M/N/53 or the control A/M adenovirus (2 x 10⁹ pfu/injection) or buffer alone (1% sucrose in PBS) were administered twice per week for a total of 8 doses/group. Tumor dimensions and body weights were measured twice per week for 7 weeks, and tumor volume was estimated as described previously. Animals were then followed to observe the effect of treatment on mouse survival.

### RESULTS

### Construction of Recombinant p53-Adenovirus

p53 adenoviruses were constructed by replacing a portion of the E1a and E1b region of adenovirus Type 5 with p53 CDNA under the control of either the Ad2 MLP (A/M/53) or CMV (A/C/53) promoter (schematized in Figure 4). This E1 substitution severely impairs the ability of the recombinant adenoviruses to replicate, restricting their propagation to 293 cells which supply Ad 5 E1 gene products in trans (Graham *et al*. (1977)). After identification of p53 recombinant adenovirus by both restriction digest and PCR analysis, the entire p53 CDNA sequence from one of the recombinant adenoviruses (A/M/53) was sequenced to verify that it was free of mutations. Following this, purified preparations of the p53 recombinants were used to infect HeLa cells to assay for the presence of phenotypically wild type adenovirus. HeLa cells, which are non-permissive for replication of E1-deleted adenovirus, were infected with 1-4 x 10⁹ infectious units of recombinant adenovirus, cultured for 3 weeks, and observed for the appearance of cytopathic effect (CPE). Using this assay, recombinant adenovirus replication or wild type contamination was not detected, readily evident by the CPE observed in control cells infected with wild type adenovirus at a level of sensitivity of approximately 1 in 10⁹.

### p53 Protein Expression from Recombinant Adenovirus

To determine if p53 recombinant adenoviruses expressed p53 protein, tumor cell lines which do not express endogenous p53 protein were infected. The human tumor cell lines Saos-2 (osteosarcoma) and Hep 3B (hepatocellular carcinoma) were infected for 24 hours with the p53 recombinant adenoviruses A/M/53 or A/C/53 at MOIs ranging 0.1 to 200 pfu/cell. Western analysis of lysates prepared from infected cells demonstrated a dose-dependent p53 protein expression in both cell types (Figure 5). Both cell lines expressed higher levels of p53 protein following infection with A/C/53 than with A/M/53 (Figure 3). No p53 protein was detected in non-infected cells. Levels of endogenous wild-type p53 are normally quite low, and nearly undetectable by Western analysis of cell extracts (Bartek *et al*. (1991)). It is clear however that wild-type p53 protein levels are easily detectable after infection with either A/M/53 or A/C/53 at the lower MOIs (Figure 5), suggesting that even low doses of p53 recombinant adenoviruses can produce potentially efficacious levels of p53.

### p53 Dependent Morphology Changes

The reintroduction of wild-type p53 into the p53-negative osteosarcoma cell line, Saos-2, results in a characteristic enlargement and flattening of these normally spindle-shaped cells (Chen *et al*. (1990)). Subconfluent Saos-2 cells (1x10⁵ cells/10cm plate) were infected at an MOI of 50 with either the A/C/53 or control A/M virus, and incubated at 37°C for 72 hours until uninfected control plates were confluent. At this point, the expected morphological change was evident in the A/C/53 treated plate (Figure 6, panel C) but not in uninfected (Figure 6, panel A) or control virus-infected plates (Figure 6, panel B). This effect was not a function of cell density because a control plate initially seeded at lower density retained normal morphology at 72 hours when its confluence approximated that of the A/C/53 treated plate. Previous results had demonstrated a high level of p53 protein expression at an MOI of 50 in Saos-2 cells (Figure 5A), and these results provided evidence that the p53 protein expressed by these recombinant adenoviruses was biologically active.

### p53 Inhibition of Cellular DNA Synthesis

To further test the activity of the p53 recombinant adenoviruses, their ability to inhibit proliferation of human tumor cells was assayed as measured by the uptake of ³H-thymidine. It has previously been shown that introduction of wild-type p53 into cells which do not express endogenous wild-type p53 can arrest the cells at the G₁/S transition, leading to inhibition of uptake of labeled thymidine into newly synthesized DNA (Baker *et al*. (1990); Mercer *et al*. (1990); Diller *et al*. (1990)). A variety of p53-deficient tumor cell lines were infected with either A/M/N/53, A/C/N/53 or a non-p53 expressing control recombinant adenovirus (A/M). A strong, dose-dependent inhibition of DNA synthesis by both the A/M/N/53 and A/C/N/53 recombinants in 7 out of the 9 different tumor cell lines tested (Figure 7) was observed. Both constructs were able to inhibit DNA synthesis in these human tumor cells, regardless of whether they expressed mutant p53 or failed to express p53 protein. It also was found that in this assay, the A/C/N/53 construct was consistently more potent than the A/M/N/53. In saos-2 (osteosarcoma) and MDA-MB468 (breast cancer) cells, nearly 100% inhibition of DNA synthesis was achieved with the A/C/N/53 construct at an MOI as low as 10. At doses where inhibition by the control adenovirus in only 10-30%, a 50-100% reduction in DNA synthesis using either p53 recombinant adenovirus was observed. In contrast, no significant p53-specific effect was observed with either construct as compared to control virus in HEP G2 cells (hepatocarcinoma cell line expressing endogenous wild-type p53, Bressac *et al*. (1990)), nor in the K562 (p53 null) leukemic cell line.

### Tumorigenicity in Nude Mice

In a more stringent test of function for the p53 recombinant adenoviruses, tumor cells were infected *ex vivo* and then injected the cells into nude mice to assess the ability of the recombinants to suppress tumor growth *in vivo.* Saos-2 cells infected with A/M/N/53 or control A/M virus at a MOI of 3 or 30, were injected into opposite flanks of nude mice. Tumor sizes were then measured twice a week over an 8 week period. At the MOI of 30, no tumor growth was observed in the p53-treated flanks in any of the animals, while the control treated tumors continued to grow (Figure 8). The progressive enlargement of the control virus treated tumors were similar to that observed in the buffer treated control animals. A clear difference in tumor growth between the control adenovirus and the p53 recombinant at the MOI of 3, although tumors from 2 out of the 4 p53-treated mice did start to show some growth after approximately 6 weeks. Thus, the A/M/N/53 recombinant adenovirus is able to mediate p53-specific tumor suppression in an *in vivo* environment.

### In Vivo Expression of Ad/p53

Although *ex vivo* treatment of cancer cells and subsequent injection into animals provided a critical test of tumor suppression, a more clinically relevant experiment is to determine if injected p53 recombinant adenovirus could infect and express p53 in established tumors *in vivo.* To address this, H69 (SCLC, p53^{null}) cells were injected subcutaneously into nude mice, and tumors were allowed to develop for 32 days. At this time, a single injection of 2 x 10⁹ pfu of either A/C/53 or A/C/β-gal adenovirus was injected into the peritumoral space surrounding the tumor. Tumors were then excised at either Day 2 or Day 7 following the adenovirus injection, and polyA RNA was isolated from each tumor. RT-PCR, using recombinant-p53 specific primers, was then used to detect p53 MRNA in the p53 treated tumors (Figure 9, lanes 1,2,4,5). No p53 signal was evident from the tumors excised from the β-gal treated animals (Figure 9, lanes 3 and 6). Amplification with actin primers served as a control for the RT-PCR reaction (Figure 9, lanes 7-9), while a plasmid containing the recombinant-p53 sequence served as a positive control for the recombinant-p53 specific band (Figure 9, lane 10). This experiment demonstrates that a p53 recombinant adenovirus can specifically direct expression of p53 mRNA within established tumors following a single injection into the peritumoral space. It also shows *in vivo* viral persistence for at least one week following infection with a p53 recombinant adenovirus.

### In Vivo Efficacy

To address the feasibility of gene therapy of established tumors, a tumor-bearing nude mouse model was used. H69 cells were injected into the subcutaneous space on the right flank of mice, and tumors were allowed to grow for 2 weeks. Mice then received peritumoral injections of buffer or recombinant virus twice weekly for a total of 8 doses. In the mice treated with buffer or control A/M virus, tumors continued to grow rapidly throughout the treatment, whereas those treated with the A/M/N/53 virus grew at a greatly reduced rate (Figure 10A). After cessation of injections, the control treated tumors continued to grow while the p53 treated tumors showed little or no growth for at least one week in the absence of any additional supply of exogenous p53 (Figure 10A). Although control animals treated with buffer alone had accelerated tumor growth as compared to either virus treated group, no significant difference in body weight was found between the three groups during the treatment period. Tumor ulceration in some animals limited the relevance of tumor size measurements after day 42. However, continued monitoring of the animals to determine survival time demonstrated a survival advantage for the p53-treated animals (Figure 10B). The last of the control adenovirus treated animals died on day 83, while buffer alone treated controls had all expired by day 56. In contrast, all 5 animals treated with the A/M/N/53 continue to survive (day 130 after cell inoculation) (Figure 10B). Together, this data establish a p53-specific effect on both tumor growth and survival time in animals with established p53-deficient tumors.

### Adenovirus Vectors Expressing p53

Recombinant human adenovirus vectors which are capable of expressing high levels of wild-type p53 protein in a dose dependent manner were constructed. Each vector contains deletions in the E1a and E1b regions which render the virus replication deficient (Challberg and Kelly (1979); Horowitz, (1991)). Of further significance is that these deletions include those sequences encoding the E1b 19 and 55 kd protein. The 19 kd protein is reported to be involved in inhibiting apoptosis (White *et al*. (1992); Rao *et al*. (1992)), whereas the 55 kd protein is able to bind wild-type p53 protein (Sarnow *et al*. (1982); Heuvel *et al*. (1990)). By deleting these adenoviral sequences, potential inhibitors of p53 function were removed through direct binding to p53 or potential inhibition of p53 mediated apoptosis. Additional constructs were made which have had the remaining 3' E1b sequence, including all protein IX coding sequence, deleted as well. Although this has been reported to reduce the packaging size capacity of adenovirus to approximately 3 kb less than wild-type virus (Ghosh-Choudhury *et al*. (1987)), these constructs are also deleted in the E3 region so that the A/M/N/53 and A/C/N/53 constructs are well within this size range. By deleting the pIX region, adenoviral sequences homologous to those contained in 293 cells are reduced to approximately 300 base pairs, decreasing the chances of regenerating replication-competent, wild-type adenovirus through recombination. Constructs lacking pIX coding sequence appear to have equal efficacy to those with pIX.

### p53/Adenovirus Efficacy In Vitro

In concordance with a strong dose dependency for expression of p53 protein in infected cells, a dose-dependent, p53-specific inhibition of tumor cell growth was demonstrated. Cell division, was inhibited and demonstrated by the inhibition of DNA synthesis, in a wide variety of tumor cell types known to lack wild-type p53 protein expression. Bacchetti and Graham (1993) recently reported p53 specific inhibition of DNA synthesis in the ovarian carcinoma cell line SKOV-3 by a p53 recombinant adenovirus in similar experiments. In addition to ovarian carcinoma, additional human tumor cell lines were demonstrated, representative of clinically important human cancers and including lines over-expressing mutant p53 protein, can also be growth inhibited by the p53 recombinants of this invention. At MOIs where the A/C/N/53 recombinant is 90-100% effective in inhibiting DNA synthesis in these tumor types, control adenovirus mediated suppression is less than 20%.

Although Feinstein *et al*. (1992) reported that re-introduction of wild-type p53 could induce differentiation and increase the proportion of cells in G₁ versus S+G₂ for leukemic K562 cells, no p53 specific effect was found in this line. Horvath and Weber (1988) have reported that human peripheral blood lymphocytes are highly nonpermissive to adenovirus infection. In separate experiments, the recombinant significantly infected the non-responding K562 cells with recombinant A/C/β-gal adenovirus, while other cell lines, including the control Hep G2 line and those showing a strong p53 effect, were readily infectable. Thus, at least part of the variability of efficacy would appear to be due to variability of infection, although other factors may be involved as well.

The results observed with the A/M/N/53 virus in Figure 8 demonstrates that complete suppression is possible in an *in vivo* environment. The resumption of tumor growth in 2 out of 4, p53 treated animals at the lower MOI most likely resulted from a small percentage of cells not initially infected with the p53 recombinant at this dose. The complete suppression seen with A/M/N/53 at the higher dose, however, shows that the ability of tumor growth to recover can be overcome.

### p53/Adenovirus In Vivo Efficacy

Work presented here and by other groups (Chen *et al*. (1990); Takahashi *et al*. (1992)) have shown that human tumor cells lacking expression of wild-type p53 can be treated *ex vivo* with p53 and result in suppression of tumor growth when the treated cells are transferred into an animal model. Applicants present the first evidence of tumor suppressor gene therapy of an *in vivo* established tumor, resulting in both suppression of tumor growth and increased survival time. In Applicants' system, delivery to tumor cells did not rely on direct injection into the tumor mass. Rather, p53 recombinant adenovirus was injected into the peritumoral space, and p53 mRNA expression was detected within the tumor. p53 expressed by the recombinants was functional and strongly suppressed tumor growth as compared to that of control, non-p53 expressing adenovirus treated tumors. However, both p53 and control virus treated tumor groups showed tumor suppression as compared to buffer treated controls. It has been demonstrated that local expression of tumor necrosis factor (TNF), interferon-γ), interleukin (IL)-2, IL-4 or IL-7 can lead to T-cell independent transient tumor suppression in nude mice (Hoch *et al*. (1992)). Exposure of monocytes to adenovirus virions are also weak inducers of IFN-α/β (reviewed in Gooding and Wold (1990)). Therefore, it is not surprising that some tumor suppression in nude mice was observed even with the control adenovirus. This virus mediated tumor suppression was not observed in the *ex vivo* control virus treated Saos-2 tumor cells described earlier. The p53-specific *in vivo* tumor suppression was dramatically demonstrated by continued monitoring of the animals in Figure 10. The survival time of the p53-treated mice was significantly increased, with 5 out of 5 animals still alive more than 130 days after cell inoculation compared to 0 out of 5 adenovirus control treated animals. The surviving animals still exhibit growing tumors which may reflect cells not initially infected with the p53 recombinant adenovirus. Higher or more frequent dosing schedules may address this. In addition, promoter shutoff (Palmer *et al*. (1991)) or additional mutations may have rendered these cells resistant to the p53 recombinant adenovirus treatment. For example, mutations in the recently described WAF1 gene, a gene induced by wild-type p53 which subsequently inhibits progression of the cell cycle into S phase, (El-Deiry *et al*. (1993); Hunter (1993)) could result in a p53-resistant tumor.

### EXPERIMENT NO. III

This Example shows the use of suicide genes and tissue specific expression of such genes in the gene therapy methods described herein. Hepatocellular carcinoma was chosen as the target because it is one of the most common human malignancies affecting man, causing an estimated 1,250,000 deaths per year world-wide. The incidence of this cancer is very high in Southeast Asia and Africa where it is associated with Hepatitis B and C infection and exposure to aflatoxin. Surgery is currently the only treatment which offers the potential for curing HCC, although less than 20% of patients are considered candidates for resection (Ravoet C. et al., 1993). However, tumors other than hepatocellular carcinoma are equally applicable to the methods of reducing their proliferation described herein.

### CELL LINES

All cell lines but for the HLF cell line were obtained from the American Type Tissue Culture Collection (ATCC) 12301 Parklawn Drive, Rockville Maryland. ATCC accession numbers are noted in parenthesis. The human embryonal kidney cell line 293 (CRL 1573) was used to generate and propagate the recombinant adenoviruses described herein. They were maintained in DME medium containing 10% defined, supplemented calf serum (Hyclone). The hepatocellular carcinoma cell lines Hep 3B (HB 8064), Hep G2 (HB 8065), and HLF were maintained in DME/F12 medium supplemented with 10% fetal bovine serum, as were the breast carcinoma cell lines MDA-MB468 (HTB 132) and BT-549 (HTB 122). Chang liver cells (CCL 13) were grown in MEM medium supplemented with 10% fetal bovine serum. The HLF cell line was obtained from Drs. T. Morsaki and H. Kitsuki at the Kyushu University School of Medicine in Japan.

### RECOMBINANT VIRUS CONSTRUCTION

Two adenoviral expression vectors designated herein as ACNTK and AANTK and devoid of protein IX function (depicted in Figure 11) are capable of directing expression of the TK suicide gene within tumor cells. A third adenovirus expression vector designated AANCAT was constructed to further demonstrate the feasibility of specifically targeting gene expression to specific cell types using adenoviral vectors. These adenoviral constructs were assembled as depicted in Figures 11 and 12 and are derivatives of those previously described for the expression of tumor suppresor genes.

For expression of the foreign gene, expression cassettes have been inserted that utilize either the human cytomegalovirus immediate early promoter/enhancer (CMV) (Boshart, M. et al., 1985) or the human alpha-fetoprotein (AFP) enhancer/promoter (Watanable, K. et al., 1987; Nakabayashi, H. et al., 1989) to direct transcription of the TK gene or the chloramphenicol acetyltransferase gene (CAT). The CMV enhancer promoter is capable of directing robust gene expression in a wide variety of cell types while the AFP enhancer/promoter construct restricts expression to hepatocellular carcinoma cells (HCC) which express AFP in about 70-80% of the HCC pateint population. In the construct utilizing the CMV promoter/enhancer, the adenovirus type 2 tripartite leader sequence also was inserted to enhance translation of the TK transcript (Berkner, K.L. and Sharp, 1985). In addition to the E1 deletion, both adenovirus vectors are additionally deleted for 1.9 kilobases (kb) of DNA in the viral E3 region. The DNA deleted in the E3 region is non-essential for virus propagation and its deletion increases the insert capacity of the recombinant virus for foreign DNA by an equivalent amount (1.9kb) (Graham and Prevec, 1991).

To demonstrate the specificity of the AFP promoter/enhancer, the virus AANCAT also was constructed where the marker gene chloramphenicol aceytitransferase (CAT) is under the control of the AFP enhancer/promoter. In the ACNTK viral construct, the Ad2 tripartite leader sequence was placed between the CMV promoter/enhancer and the TK gene. The tripartite leader has been reported to enhance translation of linked genes. The E1 substitution impairs the ability of the recombinant viruses to replicate, restricting their propagation to 293 cells which supply the Ad5 E1 gene products in trans (Graham et al., 1977).

Adenoviral Vector ACNTK: The plasmid pMLBKTK in E. coli HB101 (from ATCC #39369) was used as the source of the herpes simplex virus (HSV-1) thymidine kinase (TK) gene. TK was excised from this plasmid as a 1.7 kb gene fragment by digestion with the restriction enzymes Bgl II and Pvu II and subcloned into the compatible Bam HI, EcoR V restriction sites of plasmid pSP72 (Promega) using standard cloning techniques (Sambrook et al., 1989). The TK insert was then isolated as a 1.7 kb fragment from this vector by digestion with Xba I and Bgl II and cloned into Xba I, BamHI digested plasmid pACN (Wills et al. 1994). Twenty (20) µg of this plasmid designated pACNTK were linearized with Eco RI and cotransfected into 293 cells (ATCC CRL 1573) with 5 µg of Cla I digested ACBGL (Wills et al., 1994 supra) using a CaPO₄ transfection kit (Stratagene, San Diego, California). Viral plaques were isolated and recombinants, designated ACNTK, were identified by restriction digest analysis of isolated DNA with Xho I and BsiWI. Positive recombinants were further purified by limiting dilution and expanded and titered by standard methods (Graham and Prevec, 1991).

Adenoviral Vector AANTK: The α-fetoprotein promoter (AFP-P) and enhancer (AFP-E) were cloned from a human genomic DNA (Clontech) using PCR amplification with primers containing restriction sites at their ends. The primers used to isolate the 210 bp AFP-E contained a Nhe I restriction site on the 5' primer and an Xba I, Xho I, Kpn I linker on the 3' primer. The 5' primer sequence was 5'-CGC GCT AGC TCT GCC CCA AAG AGC T-3. The 5' primer sequence was 5'-CGC GGT ACC CTC GAG TCT AGA TAT TGC CAG TGG TGG AAG-3'. The primers used to isolate the 1763 bp AFE fragment contained a Not I restriction site on the 5' primer and a Xba I site on the 3' primer. The 5' primer sequence was 5'-CGT GCG GCC GCT GGA GGA CTT TGA GGA TGT CTG TC-3'. The 3' primer sequence was 5'-CGC TCT AGA GAG ACC AGT TAG GAA GTT TTC GCA-3'. For PCR amplification, the DNA was denatured at 97° for 7 minutes, followed by 5 cycles of amplification at 97°, 1 minute, 53°, 1 minute, 72°, 2 minutes, and a final 72°, 10 minute extension. The amplified AFE was digested with Not I and Xba I and inserted into the Not I, Xba I sites of a plasmid vector (pA/ITR/B) containing adenovirus type 5 sequences 1-350 and 3330 - 5790 separated by a polylinker containing Not I, Xho I, Xba I, Hind III, Kpn I, Bam HI, Nco I, Sma I, and Bgl II sites. The amplified AFP-E was digested with Nhe I and Kpn I and inserted into the AFP-E containing construct described above which had been digested with Xba I and Kpn I. This new construct was then further digested with Xba I and NgoMI to remove adenoviral sequences 3330 - 5780, which were subsequently replaced with an Xba I, NgoMI restriction fragment of plasmid pACN containing nucleotides 4021 - 10457 of adenovirus type 2 to construct the plasmid pAAN containing both the α-fetoprotein enhancer and promoter. This construct was then digested with Eco RI and Xba I to isolate a 2.3 kb fragment containing the Ad5 inverted terminal repeat, the AFP-E and the AFP-P which was subsequently ligated with the 8.55 kb fragment of Eco RI, Xba I digested pACNTK described above to generate pAANTK where the TK gene is driven by the α-fetoprotein enhancer and promoter in an adenovirus background. This plasmid was then linearized with Eco RI and cotransfected with the large fragment of Cla I digested ALBGL as above and recombinants, designated AANTK, were isolated and purified as described above.

Adenoviral Vector AANCAT: The chloramphenicol acetyltransferase (CAT) gene was isolated from the pCAT-Basic Vector (Promega Corporation) by an Xba I, Bam HI digest. This 1.64 kb fragment was ligated into Xba I, Bam HI digested pAAN (described above) to create pAANCAT. This plasmid was then linearized with Eco RI and cotransfected with the large fragment of Cla I digested rA/C/β-gal to create AANCAT.

### REPORTER GENE EXPRESSION: β-GALACTOSIDASE EXPRESSION:

Cells were plated at 1 x 10⁵ cells/well in a 24-well tissue culture plate (Costar) and allowed to adhere overnight (37C, 7% CO₂). Overnight infections of ACBGL were performed at a multiplicity of infection (MOI) of 30. After 24 hours, cells were fixed with 3.7% Formaldehyde; PBS, and stained with 1mg/ml Xgal reagent (USB). The data was scored (+,++,+++) by estimating the percentage of positively stained cells at each MOI. [+=1-33%, ++=33-67% and +++=>67%]

### REPORTER GENE EXPRESSION: CAT EXPRESSION:

Two (2) x 10⁶ cells (Hep G2, Hep 3B, HLF, Chang, and MDA-MB468) were seeded onto 10 cm plates in triplicate and incubated overnight (37C, 7% CO₂). Each plate was then infected with either AANCAT at an MOI = 30 or 100 or uninfected and allowed to incubate for 3 days. The cells were then trypsinized and washed with PBS and resuspended in 100 µl of 0.25 M Tris pH 7.8. The samples were frozen and thawed 3 times, and the supernatant was transferred to new tubes and incubated at 60°C for 10 minutes. The samples were then spun at 4°C for 5 minutes, and the supernatants assayed for protein concentration using a Bradford assay (Bio-Rad Protein Assay Kit). Samples were adjusted to equal protein concentrations to a final volume of 75 µl using 0.25 M Tris, 25 µl of 4mM acetyl CoA and 1 µl of ¹⁴C-Chloramphenicol and incubated overnight at 37°C. 500 µl of ethyl acetate is added to each sample and mixed by vortexing, followed by centrifiguration for 5 minutes at room temperature. The upper phase is then transferred to a new tube and the ethyl acetate is evaporated by centrifugation under vacuum. The reaction products are then redissolved in 25 µl of ethyl acetate and spotted onto a thin layer chromatography (TLC) plate and the plate is then placed in a pre-equilibrated TLC chamber (95% chloroform, 5% methanol). The solvent is then allowed to migrate to the top of the plate, the plate is then dried and exposed to X-ray film.

### CELLULAR PROLIFERATION: ³H-THYMIDINE INCORPORATION

Cells were plated at 5 x 10³ cells/well in a 96-well micro-titer plate (Costar) and allowed to incubate overnight (37C, 7% CO₂). Serially diluted ACN, ACNTK or AATK virus in DMEM; 15% FBS; 1% glutamine was used to transfect cells at an infection multiplicity of 30 for an overnight duration at which point cells were dosed in triplicate with ganciclovir (Cytovene) at log intervals betweem 0.001 and 100 mM (micro molar). 1 µCi ³H-thymidine (Amersham) was added to each well 12-18 hours before harvesting. At 72 hours-post infection cells were harvested onto glass-fiber filters and incorporated ³H-thymidine was counted using liquid scintillation (TopCount, Packard). Results are plotted as percent of untreated control proliferation and tabulated as the effective dose (ED₅₀±SD) for a 50 percent reduction in proliferation over media controls. ED₅₀ values were estimated by fitting a logistic equation to the dose response data.

### CYTOTOXICITY: LDH RELEASE

Cells (HLF, human HCC) were plated, infected with ACN or ACNTK and treated with ganciclovir as described for the proliferation assay. At 72 hours post-ganciclovir administration, cells were spun, the supernatant was removed. The levels of lactate dehydrogenase measured colometrically (Promega, Cytotox 96™). Mean (+/- S.D.) LDH release is plotted versus M.O.I.

### IN VIVO THERAPY

Human hepatocellular carcinoma cells (Hep 3B) were injected subcutaneously into ten female (10) athymic nu/nu mice (Simonsen Laboratories, Gilroy, CA). Each animal received approximately 1 x 10⁷ cells in the left flank. Tumors were allowed to grow for 27 days before randomizing mice by tumor size. Mice were treated with intratumoral and peritumoral injections of ACNTK or the control virus ACN (1 x 10⁹ iu in 100 µl) every other day for a total of three doses. Starting 24 hours after the initial dose of adenovirus, the mice were dosed intraperitoneally with ganciclovir (Cytovene 100 mg/kg) daily for a total of 10 days. Mice were monitored for tumor size and body weight twice weekly. Measurements on tumors were made in three dimensions using vernier calipers and volumes were calculated using the formula 4/3 π r³, where r is one-half the average tumor dimension.

### RESULTS

The recombinant adenoviruses were used to infect three HCC cell lines (HLF, Hep3B and Hep-G2). One human liver cell line (Chang) and two breast cancer cell lines were used as controls (MDAMB468 and BT549). To demonstrate the specificity of the AFP promoter/enhancer, the virus AANCAT was constructed. This virus was used to infect cells that either do (Hep 3B, HepG2) or do not (HLE, Chang, MDAMB468) express the HCC tumor marker alpha-fetoprotein (AFP). As shown in Figure 13, AANCAT directs expression of the CAT marker gene only in those HCC cells which are capable of expressing AFP (Figure 13).

The efficacy of ACNTK and AANTK for the treatment of HCC was assessed using a ³H-thymidine incorporation assay to measure the effect of the combination of HSV-TK expression and ganciclovir treatment upon cellular proliferation. The cell lines were infected with either ACNTK or AANTK or the control virus ACN (Wills et al., 1994 supra), which does not direct expression of HSV-TK, and then treated with increasing concentrations of ganciclovir. The effect of this treatment was assessed as a function of increasing concentrations of ganciclovir, and the concentration of ganciclovir required to inhibit ³H-thymidine incorporated by 50% was determined (ED₅₀). Additionally, a relative measure of adenovirus - mediated gene transfer and expression of each cell line was determined using a control virus which directs expression of the marker gene beta-galactosidase. The data presented in Figure 14 and Table 1 below show that the ACNTK virus/ganciclovir combination treatment was capable of inhibiting cellular proliferation in all cell lines examined as compared with the control adenovirus ACN in combination with ganciclovir. In contrast, the AANTK viral vector was only effective in those HCC cell lines which have been demonstrated to express α-fetoprotein. In addition, the AANTK/GCV combination was more effective when the cells were plated at high densities.

**TABLE 1**

| Cell Line | aFP | β-gal Expression | ACN | ED50 ACNTK | AANTK |
|---|---|---|---|---|---|
| MDAMB468 | - | +++ | >100 | 2 | >100 |
| BT549 | - | +++ | >100 | <0.3 | >100 |
| HLF | - | +++ | >100 | 0.8 | >100 |
| CHANG | - | +++ | >100 | 22 | >100 |
| HEP-3B | - | + | 80 | 8 | 8 |
| HEP-G2 LOW | + | ++ | 90 | 2 | 35 |
| HEP-G2 HIGH | + | ++ | 89 | 0.5 | 4 |

Nude mice bearing Hep3B tumors (N=5/group) were treated intratumorally and peritumorally with equivalent doses of ACNTK or ACN control. Twenty-four hours after the first administration of recombinant adenovirus, daily treatment of ganciclovir was initiated in all mice. Tumor dimensions from each animal were measured twice weekly via calipers, and average tumor sizes are plotted in Figure 16. Average tumor size at day 58 was smaller in the ACNTK-treated animals but the difference did not reach statistical significance (p<0.09, unpaired t-test). These data support a specific effect of ACNTK on tumor growth in vivo. No significant differences in average body weight were detected between the groups.

### REFERENCES

AIELLO, L. *et al*. (1979) Virology **94**:460-469.
AMERICAN CANCER SOCIETY. (1993) Cancer Facts and Figures.
AULITZKY *et al*. (1991) Eur. J. Cancer **27**(4) :462-467.
AUSTIN, E.A. and HUBER, B.E. (1993) Mol. Pharmaceutical **43**:380-387.
BACCHETTI, S. AND GRAHAM, F. (1993) International Journal of Oncology **3**:781-788.
BAKER S.J., MARKOWITZ, S., FEARON E.R., WILLSON, J.K.V., AND VOGELSTEIN, B. (1990) Science **249**:912-915.
BARTEK, J., BARTKOVA, J., VOJTESEK, B., STASKOVA, Z., LUKAS, J., REJTHAR, A., KOVARIK, J., MIDGLEY, C.A., GANNON, J.V., AND LANE, D.P. (1991) Oncogene **6**:1699-1703.
BERKNER, K.L. and SHARP (1985) Nucleic Acids Res **13**:841-857.
BOSHART, M. *et al*. (1985) Cell **41**:521-530.
BRESSAC, B., GALVIN, K.M., LIANG, T.J., ISSELBACHER, K.J., WANDS, J.R., AND OZTURK, M. (1990) Proc. Natl. Acad. Sci. USA **87**:1973-1977.
CARUSO M. *et al*. (1993) Proc. Natl. Acad. Sci. USA **90**:7024-7028.
CHALLBERG, M.D., KELLY, T.J. (1979) Biochemistry **76**:655-659.
CHEN P.L., CHEN Y., BOOKSTEIN R., AND LEE W.H. (1990) Science **250**:1576-1580.
CHEN, Y., CHEN, P.L., ARNAIZ, N., GOODRICH, D., AND LEE, W.H. (1991) Oncogene **6**:1799-1805.
CHENG, JL, YEE, J.K., YEARGIN, J., FRIEDMANN, T., AND HAAS, M. (1992) Cancer Research **52**:222-226.
COLBY, W.W. AND SHENK, T. J. (1981) Virology **39**:977-980.
CULVER ET AL. (1991) P.N.A.S. (U.S.A.) **88**:3155-3159.
CULVER, K.W. *et al*. (1992) Science **256**:1550-1552.
DEMETRI *et al*. (1989) J. Clin. Oncol. **7**(10):1545-1553.
DILLER, L., *et al*. (1990) Mol. Cell. Biology **10**:5772-5781.
EL-DEIRY, W.S., *et al*. (1993) Cell **75**:817-825.
EZZIDINE, Z.D. *et al*. (1991) The New Biologist **3**:608-614.
FEINSTEIN, E., GALE, R.P., REED, J., AND CANAANI, E. (1992) Oncogene **7**:1853-1857.
GHOSH-CHOUDHURY, G., HAJ-AHMAD, Y., AND GRAHAM, F.L. (1987) EMBO Journal **6**:1733-1739.
GOODING, L.R., AND WOLD, W.S.M. (1990) Crit. Rev. Immunol. **10**:53-71.
GRAHAM F.L., AND VAN DER ERB A.J. (1973) Virology **52**:456-467.
GRAHAM, F.L. AND PREVEC, L. (1992) Vaccines: New Approaches to Immunological Problems. R.W. Ellis (ed), Butterworth-Heinemann, Boston. pp. 363-390.
GRAHAM, F.L., SMILEY, J., RUSSELL, W.C. AND NAIRN, R. (1977) J. Gen. Virol. **36**:59-74.
GRAHAM F.L. AND PREVEC L. (1991) Manipulation of adenovirus vectors. In: Methods in Molecular Biology. Vol 7: Gene Transfer and Expression Protocols. Murray E.J. (ed.) The Humana Press Inc., Clifton N.J., Vol **7**:109-128.
HEUVEL, S.J.L., LAAR, T., KAST, W.M., MELIEF, C.J.M., ZANTEMA, A., AND VAN DER EB, A.J. (1990) EMBO Journal **9**:2621-2629.
HOCK, H., DORSCH, M., KUZENDORF, U., QIN, Z., DIAMANTSTEIN, T., AND BLANKENSTEIN, T. (1992) Proc. Natl. Acad. Sci. USA **90**:2774-2778.
HOLLSTEIN, M., SIDRANSKY, D., VOGELSTEIN, B., AND HARRIS, C. (1991) Science **253**:49-53.
HOROWITZ, M.S. (1991) Adenoviridae and their replication. In Fields Virology. B.N. Fields, ed. (Raven Press, New York) pp. 1679-1721.
HORVATH, J., AND WEBER, J.M. (1988) J. Virol. **62**:341-345.
HUANG *et al*. (1991) Nature **350**:160-162.
HUBER, B.E. *et al*. (1991) Proc. Natl. Acad. Sci. USA **88**:8039-8043.
HUNTER, T. (1993) Cell **75**:839-841.
JONES, N. AND SHENK, T. (1979) Cell **17**:683-689.
KAMB *et al*. (1994) Science **264**:436-440.
KEURBITZ, S.J., PLUNKETT, B.S., WALSH, W.V., AND KASTAN, M.B. (1992) Proc. Natl. Acad. Sci. USA **89**: 7491-7495.
KREIGLER, M. Gene Transfer and Expression: A Laboratory Manual, W.H. Freeman and Company, New York (1990).
LANDMANN *et al*. (1992) J. Interferon Res. **12**(2):103-111.
LANE, D.P. (1992) Nature **358**:15-16.
LANTZ *et al*. (1990) Cytokine **2**(6):402-406.
LARRICK, J.W. and BURCK, K.L. Gene Therapy: Application of Molecular Biology, Elsevier Science Publishing Co., Inc. New York, New York (1991).
LEE *et al*. (1987) Science **235**:1394-1399.
LEMAISTRE *et al*. (1991) Lancet **337**:1124-1125.
LEMARCHAND, P., *et al*. (1992) Proc. Natl. Acad. Sci. USA **89**:6482-6486.
LEVINE, A.J. (1993) The Tumor Suppressor Genes. Annu. Rev. Biochem. 1993. **62**:623-651.
LOWE S.W., SCHMITT, E.M., SMITH, S.W., OSBORNE, B.A., AND JACKS, J. (1993) Nature **362**:847-852.
LOWE, S.W., RULEY, H.E., JACKS, T., AND HOUSMAN, D.E. (1993) Cell **74**:957-967.
MARTIN (1975) In: Remington's Pharm. Sci., 15th Ed. (Mack Publ. Co., Easton).
MERCER, W.E., *et al*. (1990) Proc. Natl. Acad. Sci. USA **87**:6166-6170.
NAKABAYASHI, H. *et al*. (1989) The Journal of Biological Chemistry **264:**266-271.
PALMER, T.D., ROSMAN, G.J., OSBORNE, W.R., AND MILLER, A.D. (1991) Proc. Natl. Acad. Sci USA **88**:1330-1334.
RAO, L., DEBBAS, M., SABBATINI, P., HOCKENBERY, D., KORSMEYER, S., AND WHITE, E. (1992) Proc. Natl. Acad. Sci. USA **89**:7742-7746.
RAVOET C. *et al*. (1993) Journal of Surgical Oncology Supplement **3**:104-111.
RICH, D.P., *et al*. (1993) Human Gene Therapy **4**:460-476.
ROSENFELD, M.A., *et al*. (1992) Cell **68**:143-155.
SAMBROOK J., FRITSCH E.F., AND MANIATIS T. (1989). Molecular Cloning: A Laboratory Manual. (Cold Spring Harbor Laboratory Press, Cold Spring Harbor).
SARNOW, P., HO, Y.S., WILLIAMS, J., AND LEVINE, A.J. (1982) Cell **28**:387-394.
SHAW, P., BOVEY, R., TARDY, S., SAHLI, R., SORDAT, B., AND COSTA, J. (1992) Proc. Natl. Acad. Sci. USA **89**:4495-4499.
SIEGFRIED, W. (1993) Exp. Clin. Endocrinol. **101**:7-11.
SORSCHER, E.J. *et al*. (1994) Gene Therapy **1**:233-238.
SPECTOR, D.J. (1983) Virology **130**:533-538.
STEWART, P.L. *et al*. (1993) EMBO Journal **12**:2589-2599.
STRAUS. S.E. (1984) Adenovirus infections in humans. In: The Adenoviruses, Ginsberg HS, ed. New York: Plenum Press, 451-496.
SUPERSAXO *et al*. (1988) Pharm. Res. **5** (8):472-476.
TAKAHASHI, T., *et al*. (1989) Science **246:** 491-494.
TAKAHASHI, T., *et al*. (1992) Cancer Research **52**:2340-2343.
THIMMAPPAYA, B. *et al*. (1982) Cell **31**:543-551.
WANG, A.M., DOYLE, M.V., AND MARK, D.F. (1989) Proc. Natl. Acad. Sci USA **86**:9717-9721.
WATANABLE, K. *et al*. (1987) The Journal of Biological Chemistry **262:**4812-4818.
WHITE, E., *et al*. (1992) Mol. Cell. Biol. **12**:2570-2580.
WILLS, K.N. *et al*. (1994) Hum. Gen. Ther. **5**:1079-1088.
YONISH-ROUACH, E., *et al*. (1991) Nature **352**:345-347.

## Claims

1. A pharmaceutical composition comprising a recombinant adenovirus expression vector, the vector comprising a gene encoding a foreign protein and a total deletion of the coding sequence of protein IX.

2. The pharmaceutical composition of claim 1, further comprising deletion of a DNA sequence designated adenovirus E1a and E1b.

3. The pharmaceutical composition of claim 1 or 2, further comprising deletion of a DNA sequence in early region 3 and/or early region 4.

4. The pharmaceutical composition of any one of claims 1 to 3, further comprising a deletion of up to forty nucleotides positioned 3' to the protein IX deletion and a foreign DNA molecule encoding a polyadenylation signal.

5. The pharmaceutical composition of any one of claims 1 to 4, wherein the adenovirus is a Group C adenovirus selected from a serotype 1, 2, 5 or 6.

6. The pharmaceutical composition of any one of claims 1 to 5, wherein the gene is a DNA molecule up to 2.6 kilobases.

7. The pharmaceutical composition of any one of claims 1 to 5, wherein the gene is a DNA molecule up to 4.5 kilobases.

8. The pharmaceutical composition of any one of claims 1 to 7, wherein the gene encodes a functional protein, a non-functional protein, or a protein fragment thereof.

9. The pharmaceutical composition of claim 8, wherein the functional protein is a tumor suppressor protein.

10. The pharmaceutical composition of claim 8, wherein the functional protein is a suicide protein.

11. A kit for reducing the proliferation of tumor cells, said kit comprising a pharmaceutical composition of any one of claims 1 to 10, a therapeutic agent selected from the group consisting of ganciclovir, 6-methoxypurine arabinonucleoside, 5-fluorouracil, 6-methyl-purine-2'-deoxyribonucleoside, and combinations thereof, and one or more pharmaceutically acceptable carriers.

12. A recombinant adenovirus expression vector comprising a gene encoding a foreign protein and a total deletion of the coding sequence of protein IX.

13. The recombinant adenovirus expression vector of claim 12, further comprising deletion of a DNA sequence designated adenovirus E1a and E1b.

14. The recombinant adenovirus expression vector of claim 12 or 13, further comprising deletion of a DNA sequence in early region 3 and/or early region 4.

15. The recombinant adenovirus expression vector of any one of claims 12 to 14, further comprising a deletion of up to forty nucleotides positioned 3' to the protein IX deletion and a foreign DNA molecule encoding a polyadenylation signal.

16. The recombinant adenovirus expression vector of any one of claims 12 to 15, wherein the adenovirus is a Group C adenovirus selected from a serotype 1, 2, 5 or 6.

17. The recombinant adenovirus expression vector of any one of claims 12 to 16, wherein the gene is a DNA molecule up to 2.6 kilobases.

18. The recombinant adenovirus expression vector of any one of claims 12 to 16, wherein the gene is a DNA molecule up to 4.5 kilobases.

19. The recombinant adenovirus expression vector of any one of claims 12 to 18, wherein the gene encodes a functional protein, a non-functional protein, or a protein fragment thereof.

20. The recombinant adenovirus expression vector of claim 19, wherein the functional protein is a tumor suppressor protein.

21. The recombinant adenovirus expression vector of claim 19, wherein the functional protein is a suicide protein.

22. The use of the recombinant adenovirus of any one of claims 20 to 21 for the preparation of a pharmaceutical composition for treating cancer, which pharmaceutical composition further comprises one or more pharmaceutically acceptable carriers.

23. The use of claim 22, wherein the cancer is associated with a lack of endogenous tumor suppressor protein.

24. The use of claim 23, wherein the cancer is non-small cell lung cancer, small cell lung cancer, hepatocarcinoma, melanoma, retinoblastoma, breast tumor, colorectal carcinoma, leukemia, lymphoma, brain tumor, cervical carcinoma, sarcoma, prostate tumor, bladder tumor, tumor of the reticulo-endothelial tissues, Wilms' tumor, astrocytoma, glioblastoma, neuroblastoma, ovarian carcinoma, osteosarcoma, and renal cancer.

25. The use of the recombinant adenovirus of any one of claims 20 to 21 for the preparation of a pharmaceutical composition for inhibiting the proliferation of a tumor in an animal, which pharmaceutical composition further comprises one or more pharmaceutically acceptable carriers.

26. The use of the recombinant adenovirus of any one of claims 20 to 21 for the preparation of a pharmaceutical composition for reducing the proliferation of tumor cells in a subject, wherein the pharmaceutical composition further comprises an effective amount of a therapeutic agent selected from the group consisting of ganciclovir, 6-methoxypurine arabinonucleoside, 5-fluorouracil, 6-methylpurine-2'-deoxyribonucleoside, and combinations thereof, and one or more pharmaceutically acceptable carriers.

27. The use of claim 26, wherein the tumor cells are hepatocellular carcinoma.

## Patentansprüche

1. Pharmazeutische Zusammensetzung, umfassend einen rekombinanten Adenovirus-Expressionsvektor, wobei der Vektor ein Gen, das ein Fremdprotein kodiert, und eine vollständige Deletion der kodierenden Sequenz von Protein IX umfaßt.

2. Pharmazeutische Zusammensetzung nach Anspruch 1, ferner umfassend Deletion einer DNA-Sequenz, die als Adenovirus E1a und E1b bezeichnet ist.

3. Pharmazeutische Zusammensetzung nach einem der Ansprüche 1 oder 2, ferner umfassend Deletion einer DNA-Sequenz in der frühen Region 3 und/oder der frühen Region 4.

4. Pharmazeutische Zusammensetzung nach irgendeinem der Ansprüche 1 bis 3, ferner umfassend Deletion von bis zu 40 Nukleotiden, die 3' zu der Protein IX-Deletion positioniert sind, und eine Fremd-DNA, die ein Polyadenylierungssignal kodiert.

5. Pharmazeutische Zusammensetzung nach irgendeinem der Ansprüche 1 bis 4, wobei das Adenovirus ein Gruppe C-Adenovirus ist, das aus den Serotypen 1, 2, 5 oder 6 ausgewählt ist.

6. Pharmazeutische Zusammensetzung nach irgendeinem der Ansprüche 1 bis 5, wobei das Gen ein DNA-Molekül mit bis zu 2,6 Kilobasen ist.

7. Pharmazeutische Zusammensetzung nach irgendeinem der Ansprüche 1 bis 5, wobei das Gen ein DNA-Molekül mit bis zu 4,5 Kilobasen ist.

8. Pharmazeutische Zusammensetzung nach irgendeinem der Ansprüche 1 bis 7, wobei das Gen ein funktionelles Protein, ein nicht-funktionelles Protein oder ein Proteinfragment davon kodiert.

9. Pharmazeutische Zusammensetzung nach Anspruch 8, wobei das funktionelle Protein ein Tumorsuppressor-Protein ist.

10. Pharmazeutische Zusammensetzung nach Anspruch 8, wobei das funktionelle Protein ein Suizid-Protein ist.

11. Kit zur Reduzierung der Proliferation von Tumorzellen, wobei das Kit eine pharmazeutische Zusammensetzung nach einem der Ansprüche 1 bis 10, ein therapeutisches Agens, ausgewählt aus der Gruppe bestehend aus Ganciclovir, 6-Methoxypurinarabinonukleosid, 5-Fluoruracil, 6-Methylpurin-2'-desoxyribonukleosid und Kombinationen davon und einen oder mehrere pharmazeutisch akzeptable Träger umfaßt.

12. Rekombinanter Adenovirus-Expressionsvektor, umfassend ein Gen, das ein Fremdprotein kodiert, und eine vollständige Deletion der kodierenden Sequenz von Protein IX.

13. Rekombinanter Adenovirus-Expressionsvektor nach Anspruch 12, ferner umfassend Deletion einer DNA-Sequenz, die als Adenovirus E1a und E1b bezeichnet ist.

14. Rekombinanter Adenovirus-Expressionsvektor nach Anspruch 12 oder 13, ferner umfassend Deletion der DNA-Sequenz in der frühen Region 3 und/oder der frühen Region 4.

15. Rekombinanter Adenovirus-Expressionsvektor nach irgendeinem der Ansprüche 12 bis 14, ferner umfassend eine Deletion von bis zu 40 Nukleotiden, die 3' zu der Protein IX-Deletion positioniert sind, und eine Fremd-DNA, die ein Polyadenylierungssignal kodiert.

16. Rekombinanter Adenovirus-Expressionsvektor gemäß einem der Ansprüche 12 bis 15 wobei der Adenovirus ein Gruppe C-Adenovirus ist, das aus den Serotypen 1, 2, 5 oder 6 ausgewählt ist.

17. Rekombinanter Adenovirus-Expressionsvektor nach irgendeinem der Ansprüche 12 bis 16, wobei das Gen ein DNA-Molekül mit bis zu 2,6 Kilobasen ist.

18. Rekombinanter Adenovirus-Expressionsvektor nach irgendeinem der Ansprüche 12 bis 16, wobei das Gen ein DNA-Molekül mit bis zu 4,5 Kilobasen ist.

19. Rekombinanter Adenovirus-Expressionsvektor nach irgendeinem der Ansprüche 12 bis 18, wobei das Gen ein funktionelles Protein, ein nicht-funktionelles Protein oder ein Proteinfragment davon kodiert.

20. Rekombinanter Adenovirus-Expressionsvektor nach Anspruch 19, wobei das funktionelle Protein ein Tumorsuppressor-Protein ist.

21. Rekombinanter Adenovirus-Expressionsvektor nach Anspruch 19, wobei das funktionelle Protein ein Suizid-Protein ist.

22. Verwendung des rekombinanten Adenovirus nach irgendeinem der Ansprüche 20 bis 21 zur Herstellung einer pharmazeutischen Zusammensetzung zur Behandlung von Krebs, wobei die pharmazeutische Zusammensetzung ferner einen oder mehrere pharmazeutisch verträgliche Träger umfaßt.

23. Verwendung nach Anspruch 22, wobei der Krebs mit dem Fehlen eines endogenen Tumorsuppressor-Proteins assoziiert ist.

24. Verwendung nach Anspruch 23, wobei der Krebs ein nichtkleinzelliger Lungenkrebs, kleinzelliger Lungenkrebs, Hepatokarzinom, Melanom, Retinoblastom, Brusttumor, Colorectalkarzinom, Leukämie, Lymphom, Hirntumor, Cervixkarzinom, Sarkom, Prostatatumor, Blasentumor, Tumor der Reticuloendothelialgewebe, Wilms Tumor, Astrocytom, Glioblastom, Neuroblastom, Ovarialkarzinom, Osteosarkom und Nierenkrebs ist.

25. Verwendung des rekombinanten Adenovirus nach irgendeinem der Ansprüche 20 bis 21 zur Herstellung einer pharmazeutischen Zusammensetzung zum Inhibieren der Proliferation eines Tumors in einem Tier, wobei die pharmazeutische Zusammensetzung ferner einen oder mehrere pharmazeutisch verträgliche Träger umfaßt.

26. Verwendung des rekombinanten Adenovirus nach irgendeinem der Ansprüche 20 bis 21 zur Herstellung einer pharmazeutischen Zusammensetzung zur Reduzierung der Proliferation von Tumorzellen in einem Subjekt, wobei die pharmazeutische Zusammensetzung ferner eine wirksame Menge eines therapeutischen Agens, ausgewählt aus der Gruppe bestehend aus Ganciclovir, 6-Methoxypurin-arabinonukleosid, 5-Fluoruracil, 6-Methylpurin-2'-desoxyribonukleosid und Kombinationen davon, und einen oder mehrere pharmazeutisch akzeptable Träger umfaßt.

27. Verwendung nach Anspruch 26, wobei die Tumorzellen Hepatozelluläres Karzinom sind.

## Revendications

1. Composition pharmaceutique comprenant un vecteur d'expression adénoviral recombinant, le vecteur comprenant un gène codant une protéine étrangère et une délétion totale de la séquence codante de la protéine IX.

2. Composition pharmaceutique selon la revendication 1, comprenant en outre la délétion d'une séquence d'ADN d'adénovirus désignée E1a et E1b.

3. Composition pharmaceutique selon la revendication 1 ou 2, comprenant en outre la délétion d'une séquence d'ADN dans la région précoce 3 et/ou la région précoce 4.

4. Composition pharmaceutique selon l'une quelconque des revendications 1 à 3, comprenant en outre une délétion concernant jusqu'à quarante nucléotides positionnés en 3' de la délétion de la protéine IX et une molécule d'ADN étranger codant un signal de polyadénylation.

5. Composition pharmaceutique selon l'une quelconque des revendications 1 à 4, dans laquelle l'adénovirus est un adénovirus du groupe C choisi parmi un sérotype 1, 2, 5 ou 6.

6. Composition pharmaceutique selon l'une quelconque des revendications 1 à 5, dans laquelle le gène est une molécule d'ADN contenant jusqu'à 2,6 kilobases.

7. Composition pharmaceutique selon l'une quelconque des revendications 1 à 5, dans laquelle le gène est une molécule d'ADN contenant jusqu'à 4,5 kilobases.

8. Composition pharmaceutique selon l'une quelconque des revendications 1 à 7, dans laquelle le gène code une protéine fonctionnelle, une protéine non fonctionnelle ou un fragment protéique de celle-ci.

9. Composition pharmaceutique selon la revendication 8, dans laquelle la protéine fonctionnelle est une protéine suppresseur de tumeur.

10. Composition pharmaceutique selon la revendication 8, dans laquelle la protéine fonctionnelle est une protéine suicide.

11. Kit pour réduire la prolifération des cellules tumorales, ledit kit comprenant une composition pharmaceutique selon l'une quelconque des revendications 1 à 10, un agent thérapeutique choisi dans le groupe constitué par le ganciclovir, le 6-méthoxypurine arabinonucléoside, le 5-fluorouracile, le 6-méthyl-purine-2'-désoxyribonucléoside et leurs combinaisons, et un ou plusieurs véhicules pharmaceutiquement acceptables.

12. Vecteur d'expression adénoviral recombinant comprenant un gène codant une protéine étrangère et une délétion totale de la séquence codante de la protéine IX.

13. Vecteur d'expression adénoviral recombinant selon la revendication 12, comprenant en outre la délétion d'une séquence d'ADN d'adénovirus désignée E1a et E1b.

14. Vecteur d'expression adénoviral recombinant selon la revendication 12 ou 13, comprenant en outre la délétion d'une séquence d'ADN dans la région précoce 3 et/ou la région précoce 4.

15. Vecteur d'expression adénoviral recombinant selon l'une quelconque des revendications 12 à 14, comprenant en outre une délétion concernant jusqu'à quarante nucléotides positionnés en 3' de la délétion de la protéine IX et une molécule d'ADN étranger codant un signal de polyadénylation.

16. Vecteur d'expression adénoviral recombinant selon l'une quelconque des revendications 12 à 15, dans lequel l'adénovirus est un adénovirus du groupe C choisi parmi un sérotype 1, 2, 5 ou 6.

17. Vecteur d'expression adénoviral recombinant selon l'une quelconque des revendications 12 à 16, dans lequel le gène est une molécule d'ADN contenant jusqu'à 2,6 kilobases.

18. Vecteur d'expression adénoviral recombinant selon l'une quelconque des revendications 12 à 16, dans lequel le gène est une molécule d'ADN contenant jusqu'à 4,5 kilobases.

19. Vecteur d'expression adénoviral recombinant selon l'une quelconque des revendications 12 à 18, dans lequel le gène code une protéine fonctionnelle, une protéine fonctionnelle, ou un fragment protéique de celle-ci.

20. Vecteur d'expression adénoviral recombinant selon la revendication 19, dans lequel la protéine fonctionnelle est une protéine suppresseur de tumeur.

21. Vecteur d'expression adénoviral recombinant selon la revendication 19, dans lequel la protéine fonctionnelle est une protéine suicide.

22. Utilisation de l'adénovirus recombinant selon l'une quelconque des revendications 20 à 21 pour la préparation d'une composition pharmaceutique pour traiter le cancer, laquelle composition pharmaceutique comprend en outre un ou plusieurs véhicules pharmaceutiquement acceptables.

23. Utilisation selon la revendication 22, dans laquelle le cancer est associé à un manque de protéine suppresseur de tumeur endogène.

24. Utilisation selon la revendication 23, dans laquelle le cancer est un cancer pulmonaire non à petites cellules, un cancer pulmonaire à petites cellules, un hépatocarcinome, un mélanome, un rétinoblastome, une tumeur du sein, un carcinome colorectal, une leucémie, un lymphome, une tumeur du cerveau, un carcinome cervical, un sarcome, une tumeur de la prostate, une tumeur de la vessie, une tumeur des tissus réticulo-endothéliaux, une tumeur de Wilms, un astrocytome, un glioblastome, un neuroblastome, un carcinome ovarien, un ostéosarcome et un cancer rénal.

25. Utilisation de l'adénovirus recombinant selon l'une quelconque des revendications 20 à 21 pour la préparation d'une composition pharmaceutique pour inhiber la prolifération d'une tumeur dans un animal, laquelle composition pharmaceutique comprend en outre un ou plusieurs véhicules pharmaceutiquement acceptables.

26. Utilisation de l'adénovirus recombinant selon l'une quelconque des revendications 20 à 21 pour la préparation d'une composition pharmaceutique pour réduire la prolifération de cellules tumorales chez un sujet, dans laquelle la composition pharmaceutique comprend en outre une quantité efficace d'un agent thérapeutique choisi dans le groupe constitué par le ganciclovir, le 6-méthoxypurine arabinonucléoside, le 5-fluorouracile, le 6-méthyl-purine-2'-désoxyribonucléoside et leurs combinaisons, et un ou plusieurs véhicules pharmaceutiquement acceptables.

27. Utilisation selon la revendication 26, dans laquelle les cellules tumorales sont un carcinome hépatocellulaire.
